(19) Europäisches Patentamt
European Patent Office
Office européen des brevets

(11) Veröffentlichungsnummer: **0 311 892**
**B1**

(12) # EUROPÄISCHE PATENTSCHRIFT

(45) Veröffentlichungstag der Patentschrift: **02.01.91**

(21) Anmeldenummer: **88116525.2**

(22) Anmeldetag: **06.10.88**

(51) Int. Cl.[5]: **C 07 D 417/06,**
C 07 D 277/24,
C 07 D 277/42,
C 07 D 417/10, A 01 N 43/78

(54) **Subtituierte Azolylmethylcarbinole.**

(30) Priorität: **14.10.87 DE 3734694**
**06.09.88 DE 3830240**

(43) Veröffentlichungstag der Anmeldung:
**19.04.89 Patentblatt 89/16**

(45) Bekanntmachung des Hinweises auf die Patenterteilung:
**02.01.91 Patentblatt 91/01**

(84) Benannte Vertragsstaaten:
**BE CH DE FR GB IT LI NL**

(56) Entgegenhaltungen:
**EP-A-0 046 337**
**EP-A-0 158 205**

(73) Patentinhaber: **BAYER AG**
**D-5090 Leverkusen 1 Bayerwerk (DE)**

(72) Erfinder: **Krämer, Wolfgang, Dr.**
**Rosenkranz 25**
**D-5093 Burscheid 2 (DE)**
Erfinder: **Regel, Erik, Dr.**
**Bergerheide 26**
**D-5600 Wuppertal 1 (DE)**
Erfinder: **Büchel, Karl Heinz, Prof. Dr.**
**Dabringhausener Strasse 42**
**D-5093 Burscheid (DE)**
Erfinder: **Dutzmann, Stefan, Dr.**
**Leinenweberweg 33**
**D-4000 Düsseldorf 13 (DE)**
Erfinder: **Brandes, Wilhelm, Dr.**
**Eichendorffstrasse 3**
**D-5653 Leichlingen (DE)**
Erfinder: **Lürssen, Klaus, Dr.**
**August-Kierspel-Strasse 145**
**D-5060 Bergisch-Gladbach 2 (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 311 892 B1

**Beschreibung**

Die Erfindung betrifft neue substituierte Azolylmethylcarbinole, ein Verfahren zu ihrer Herstellung sowie ihre Verwendung als Fungizide und Pflanzenwachstumsregulatoren.

Es ist bekannt, daß bestimmte substituierte Azolylmethylcarbinole gute fungizide Eigenschaften besitzen (vgl. DE—A 3 413 173 und EP—A 0 158 205). So lassen sich zum Beispiel 1-(4-Chlorphenyl)-1-(4,5-dimethyl-thiazol-2-yl)-2-(1,2,4-triazol-1-yl)-ethanol und 1-(2,4-Dichlorphenyl)-1-(4,5-dimethyl-thiazol-2-yl)-2-(1,2,4-triazol-1-yl)-ethanol zur Bekämpfung von Pilzen verwenden. Die Wirksamkeit dieser Verbindungen ist jedoch insbesondere bei niedrigen Aufwandmengen und Konzentrationen nicht in allen Anwendungsgebieten völlig zufriedenstellend. Außerdem ist über eine pflanzenwuchsregulierende Wirksamkeit dieser vorbekannten Stoffe nichts bekannt.

Weiterhin sind aus der EP—A 0 046 337 Hydroxyethyl-azole bekannt, in denen das Carbinol-Kohlenstoffatom mit einem Heterocyclus verbunden sein kann. Es werden aber keine Stoffe beschrieben, in denen an der betreffenden Stelle ein Thiazol-4-yl-Rest enthalten ist.

Es wurden nun neue substituierte Azolylmethylcarbinole der Formel

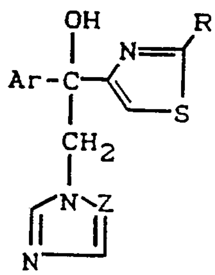

(I)

in welcher

Ar für Phenyl steht, welches einfach oder mehrfach, gleichartig oder verschieden substituiert sein kann durch Halogen, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Halogenalkoxy mit 1 bis 4 Kohlenstoffatomen und 1 bis 9 gleichen oder verschiedenen Halogenatomen und/oder durch geradkettiges oder verzweigtes Halogenalkylthio mit 1 bis 4 Kohlenstoffatomen und 1 bis 9 gleichen oder verschiedenen Halogenatomen,

Z für Stickstoff oder eine CH-Gruppe steht und

R für geradkettiges oder verzweigtes Alkyl mit 1 bis 6 Kohlenstoffatomen, geradkettiges oder verzweigtes Dialkylaminoalkyl mit 1 bis 4 Kohlenstoffatomen in jeder Alkylgruppe, geradkettiges oder verzweigtes Alkenyl mit 3 bis 6 Kohlenstoffatomen, geradkettiges oder verzweigtes Alkinyl mit 3 bis 6 Kohlenstoffatomen, geradkettiges oder verzweigtes Alkoxyalkyl mit 1 bis 6 Kohlenstoffatomen im Alkylteil und 1 bis 6 Kohlenstoffatomen im Alkoxyteil steht, oder

R für Aralkyl mit 1 bis 4 Kohlenstoffatomen im Alkylteil und 6 bis 10 Kohlenstoffatomen im Arylteil steht, wobei der Arylteil einfach oder mehrfach, gleichartig oder verschieden substituiert sein kann durch Halogen, Cyano, Nitro, Phenyl, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Halogenalkoxy mit 1 bis 4 Kohlenstoffatomen und 1 bis 9 gleichen oder verschiedenen Halogenatomen und/oder durch geradkettiges oder verzweigtes Halogenalkylthio mit 1 bis 4 Kohlenstoffatomen und 1 bis 9 gleichen oder verschiedenen Halogenatomen, oder durch einen über N verknüpften fünf- oder sechsgliedrigen Stickstoffheterocyclus, der gegebenenfalls weitere Heteroatome enthalten kann und einfach oder mehrfach, gleichartig oder verschieden substituiert sein kann durch geradkettiges oder verzweigtes Alkyl mit 1 bis 4 Kohlenstoffatomen, geradkettiges oder verzweigtes Alkoxycarbonyl mit 1 bis 4 Kohlenstoffatomen im Alkoxyteil und/oder durch Alkylcarbamoyl mit 1 bis 4 Kohlenstoffatomen im Alkylteil, oder

R für Aryloxyalkyl mit 1 bis 4 Kohlenstoffatomen im Alkylteil und 6 bis 10 Kohlenstoffatomen im Arylteil steht, wobei der Arylteil einfach oder mehrfach, gleichartig oder verschieden substituiert sein kann durch Halogen, Cyano, Nitro, Phenyl, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Halogenalkoxy mit 1 bis 4 Kohlenstoffatomen und 1 bis 9 gleichen oder verschiedenen Halogenatomen und/oder durch geradkettiges oder verzweigtes Halogenalkylthio mit 1 bis 4 Kohlenstoffatomen und 1 bis 9 gleichen oder verschiedenen Halogenatomen, oder durch einen über N verknüpften fünf- oder sechsgliedrigen Stickstoffheterocyclus, der gegebenenfalls weitere Heteroatome

enthalten kann und einfach oder mehrfach, gleichartig oder verschieden substituiert sein kann durch geradkettiges oder verzweigtes Alkyl mit 1 bis 4 Kohlenstoffatomen, geradkettiges oder verzweigtes Alkoxycarbonyl mit 1 bis 4 Kohlenstoffatomen im Alkoxyteil und/oder durch Alkylcarbamoyl mit 1 bis 4 Kohlenstoffatomen im Alkylteil, oder

R für Aryl mit 6 bis 10 Kohlenstoffatomen steht, wobei der Aryl-Rest einfach oder mehrfach, gleichartig oder verschieden substituiert sein kann durch Halogen, Cyano, Nitro, Phenyl, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Halogenalkoxy mit 1 bis 4 Kohlenstoffatomen und 1 bis 9 gleichen oder verschiedenen Halogenatomen und/oder durch geradkettiges oder verzweigtes Halogenalkylthio mit 1 bis 4 Kohlenstoffatomen und 1 bis 9 gleichen oder verschiedenen Halogenatomen, durch einen über N verknüpften fünf- oder sechsgliedrigen Stickstoffheterocyclus, der gegebenenfalls weitere Heteroatome enthalten kann und einfach oder mehrfach, gleichartig oder verschieden substituiert sein kann durch geradkettiges oder verzweigtes Alkyl mit 1 bis 4 Kohlenstoffatomen, geradkettiges oder verzweigtes Alkoxycarbonyl mit 1 bis 4 Kohlenstoffatomen im Alkoxyteil und/oder durch Alkylcarbamoyl mit 1 bis 4 Kohlenstoffatomen im Alkylteil, oder

R für einen Rest der Formel

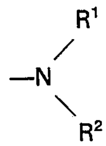

steht, worin

$R^1$ für Wasserstoff, geradkettiges oder verzweigtes Alkyl mit 1 bis 6 Kohlenstoffatomen, geradkettiges oder verzweigtes Alkenyl mit 2 bis 6 Kohlenstoffatomen, geradkettiges oder verzweigtes Alkinyl mit 2 bis 6 Kohlenstoffatomen oder für Cycloalkyl mit 3 bis 7 Kohlenstoffatomen steht und

$R^2$ für Wasserstoff, geradkettiges oder verzweigtes Alkyl mit 1 bis 16 Kohlenstoffatomen, geradkettiges oder verzweigtes Alkoxyalkyl mit 1 bis 4 Kohlenstoffatomen im Alkylteil und 1 bis 4 Kohlenstoffatomen im Alkoxyteil, geradkettiges oder verzweigtes Alkenyl mit 2 bis 6 Kohlenstoffatomen, geradkettiges oder verzweigtes Alkinyl mit 2 bis 6 Kohlenstoffatomen, Cycloalkyl mit 3 bis 7 Kohlenstoffatomen, Cycloalkylalkyl mit 3 bis 7 Kohlenstoffatomen im Cycloalkylteil und 1 bis 4 Kohlenstoffatomen im Alkylteil oder für Phenyl steht, das einfach oder mehrfach, gleichartig oder verschieden substituiert sein kann durch Halogen, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Halogenalkoxy mit 1 bis 4 Kohlenstoffatomen und 1 bis 9 gleichen oder verschiedenen Halogenatomen und/oder durch geradkettiges oder verzweigtes Halogenalkylthio mit 1 bis 4 Kohlenstoffatomen und 1 bis 9 gleichen oder verschiedenen Halogenatomen, oder

$R^2$ für Aralkyl mit 1 bis 4 Kohlenstoffatomen im Alkylteil und 6 bis 10 Kohlenstoffatomen im Arylteil steht, wobei der Arylteil einfach oder mehrfach, gleichartig oder verschieden substituiert sein kann durch Halogen, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, durch geradkettiges oder verzweigtes Halogenalkoxy mit 1 bis 4 Kohlenstoffatomen und 1 bis 9 gleichen oder verschiedenen Halogenatomen, und/oder durch geradkettiges oder verzweigtes Halogenalkylthio mit 1 bis 4 Kohlenstoffatomen und 1 bis 9 gleichen oder verschiedenen Halogenatomen, oder

$R^1$ und $R^2$ gemeinsam mit dem Stickstoffatom, an das sie gebunden sind, für einen gesättigten Heterocyclus mit 5 bis 7 Ringgliedern stehen, wobei der Heterocyclus ein weiteres Heteroatom enthalten kann und wobei der Heterocyclus einfach oder mehrfach, gleichartig oder verschieden substituiert sein kann durch geradkettiges oder verzweigtes Alkyl mit 1 bis 4 Kohlenstoffatomen, geradkettiges oder verzweigtes Hydroxyalkyl mit 1 bis 4 Kohlenstoffatomen, geradkettiges oder verzweigtes Alkanoyl mit 1 bis 4 Kohlenstoffatomen im Alkanteil und/oder durch Alkanoyloxyalkyl mit 1 bis 4 Kohlenstoffatomen im Alkanteil und 1 bis 4 Kohlenstoffatomen im Oxyalkylteil,

sowie deren Säureadditions-Salze und Metallsalz-Komplexe gefunden.

Die substituierten Azolylmethylcarbinole der Formel (I) enthalten ein asymmetrisch substituiertes Kohlenstoffatom und können deshalb in den beiden optischen Isomerenformen oder auch als Isomerengemische unterschiedlicher Zusammensetzung anfallen. Die Erfindung betrifft sowohl die Racemate als auch die einzelnen Isomeren und deren Gemische.

Weiterhin wurde gefunden, daß man die neuen substituierten Azolylmethylcarbinole der Formel (I) sowie deren Säureadditions-Salze und Metallsalz-Komplexe erhält, wenn man substituierte 2-Bromethanole der Formel

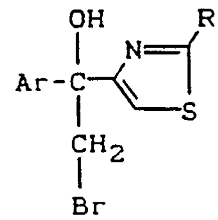

(II)

in welcher

Ar und R die angegebene Bedeutung haben,

mit Azolen der Formel

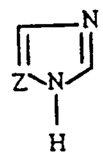

(III)

in welcher

Z die oben angegebene Bedeutung hat,

gegebenenfalls in Gegenwart eines Verdünnungsmittels und gegebenenfalls in Gegenwart eines Reaktionshilfsmittels sowie gegebenenfalls in Gegenwart eines Phasentransferkatalysators umsetzt und gegebenenfalls anschließend eine Säure oder ein Metallsalz addiert.

Schließlich wurde gefunden, daß die neuen substituierten Azolylmethylcarbinole der Formel (I) sowie deren Säureadditions-Salze und Metallsalz-Komplexe sehr gute fungizide und pflanzenwachstumsregulierende Eigenschaften besitzen.

Überraschenderweise zeigen die erfindungsgemäßen Wirkstoffe eine erheblich bessere fungizide Wirksamkeit als 1-(4-Chlorphenyl)-1-(4,5-dimethyl-thiazol-2-yl)-2-(1,2,4-triazol-1-yl)-ethanol und 1-(2,4-Dichlorphenyl)-1-(4,5-dimethyl-thiazol-2-yl)-2-(1,2,4-triazol-1-yl)-ethanol, welches konstitutionell ähnliche, vorbekannte Wirkstoffe gleicher Wirkungsrichtung sind.

Die erfindungsgemäßen substituierten Azolylmethylcarbinole sind durch die Formel (I) allgemein definiert. Bevorzugt sind Verbindungen der Formel (I), in denen

Ar für Phenyl steht, das einfach bis dreifach, gleichartig oder verschieden substituiert sein kann durch Fluor, Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy oder Trifluormethylthio,

Z für Stickstoff oder eine CH-Gruppe steht und

R für Methyl, Ethyl, n- oder i-Propyl, n-, i-, s- oder t-Butyl, Dimethylaminopropyl, Allyl, n- oder i-Butenyl, Propargyl, Methoxymethyl, Methoxyethyl, Ethoxymethyl, Ethoxyethyl, n- oder i-Propoxymethyl, n- oder i-Propoxyethyl oder für Benzyl, Phenylethyl, Phenoxymethyl oder Phenyl steht, wobei jeder dieser Benzyl-, Phenylethyl-, Phenoxymethyl- oder Phenyl-Reste einfach bis dreifach, gleichartig oder verschieden substituiert sein kann durch Fluor, Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Phenyl, gegebenenfalls ein- bis dreifach, gleich oder verschieden durch Methyl, Ethyl, Methoxycarbonyl, Ethoxycarbonyl, Methylcarbamoyl oder Ethylcarbamoyl substituiertes Triazolyl, gegebenenfalls einfach bis dreifach, gleichartig oder verschieden durch Methyl, Ethyl, Methoxycarbonyl, Ethoxycarbonyl, Methylcarbamoyl oder Ethylcarbamoyl substituiertes Imidazolyl, gegebenenfalls einfach bis dreifach, gleichartig oder verschieden durch Methyl, Ethyl, Methoxycarbonyl, Ethoxycarbonyl, Methylcarbamoyl oder Ethylcarbamoyl substituiertes Pyrazolyl, gegebenenfalls einfach bis dreifach, gleichartig oder verschieden durch Methyl, Ethyl, Methoxycarbonyl, Ethoxycarbonyl, Methylcarbamoyl oder Ethylcarbamoyl substituiertes Pyrrolidinyl, gegebenenfalls einfach bis dreifach, gleichartig oder verschieden durch Methyl, Ethyl, Methoxycarbonyl, Ethoxycarbonyl, Methylcarbamoyl oder Ethylcarbamoyl substituiertes Piperidinyl, gegebenenfalls einfach bis dreifach, gleichartig oder verschieden durch Methyl, Ethyl, Methoxycarbonyl, Ethoxycarbonyl, Methylcarbamoyl oder Ethylcarbamoyl substituiertes Morpholinyl und/oder durch gegebenenfalls einfach bis dreifach, gleichartig oder verschieden durch Methyl, Ethyl, Methoxycarbonyl, Ethoxycarbonyl, Methylcarbamoyl oder Ethylcarbamoyl substituiertes Piperazinyl, oder

R für einen Rest der Formel

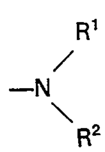

steht, worin

$R^1$ für Wasserstoff, Methyl, Ethyl, n- oder i-Propyl, n-, i-, s- oder t-Butyl, Allyl, n- oder i-Butenyl, Propargyl, n- oder i-Butinyl, Cyclopentyl oder Cyclohexyl steht und

$R^2$ für Wasserstoff, Methyl, Ethyl, n- oder i-Propyl, n-, i-, s- oder t-Butyl, Hexyl oder n-Dodecyl, Methoxymethyl, Methoxyethyl, Ethoxymethyl, Ethoxyethyl, Methoxypropyl, Ethoxypropyl, Allyl, n- oder i-Butenyl, Propargyl, n- oder i-Butinyl, Cyclopentyl, Cyclohexyl, Cyclopentyl-methyl, Cyclohexyl-methyl oder für Phenyl steht, das einfach bis dreifach, gleichartig oder verschieden substituiert sein kann durch Fluor, Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy und/oder Trifluormethylthio, oder

$R^2$ für Benzyl oder Phenethyl steht, wobei jeder dieser Reste im Phenylteil einfach bis dreifach, gleichartig oder verschieden substituiert sein kann durch Fluor, Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy und/oder Trifluormethylthio, oder

$R^1$ und $R^2$ gemeinsam mit dem Stickstoffatom, an das sie gebunden sind, für Pyrrolidinyl, Piperidinyl, Hexahydroazepinyl, Morpholinyl, Thiamorpholinyl oder 1,4-Piperazinyl stehen, wobei jeder der zuvor genannten Reste einfach bis dreifach, gleichartig oder verschieden substituiert sein kann durch Methyl, Ethyl, Hydroxymethyl, Acetyl, Propionyl und/oder Acetoxymethyl.

Ganz besonders bevorzugt sind Verbindungen der Formel (I), in denen

Ar für gegebenenfalls ein- bis zweifach, gleich oder verschieden durch Fluor, Chlor, Brom, Methyl, Methoxy, Trifluormethyl, Trifluormethoxy und/oder Trifluormethylthio substituiertes Phenyl steht,

Z für Stickstoff oder eine CH-Gruppe steht und

R für Methyl, Ethyl, n-Propyl, iso-Propyl, n-, i-, s- oder t-Butyl, Dimethylaminopropyl, Allyl, Propargyl, Methoxymethyl, Benzyl, Phenoxymethyl oder Phenyl steht, wobei jeder dieser Benzyl-, Phenoxymethyl- und Phenyl-Reste einfach oder zweifach, gleichartig oder verschieden substituiert sein kann durch Fluor, Chlor, Brom, Methyl, Methoxy, Trifluormethyl, Trifluormethoxy, Trifluormethylthio und/oder Phenyl, oder durch gegebenenfalls einfach oder zweifach, gleichartig oder verschieden durch Methyl, Methoxycarbonyl, Ethoxycarbonyl oder Methylcarbamoyl substituiertes Triazolyl, gegebenenfalls einfach oder zweifach, gleichartig oder verschieden durch Methyl, Methoxycarbonyl, Ethoxycarbonyl oder Methylcarbamoyl substituiertes Imidazolyl, gegebenenfalls einfach oder zweifach, gleichartig oder verschieden durch Methyl, Methoxycarbonyl, Ethoxycarbonyl oder Methylcarbamoyl substituiertes Pyrazolyl, gegebenenfalls einfach oder zweifach, gleichartig oder verschieden durch Methyl, Methoxycarbonyl, Ethoxycarbonyl oder Methylcarbamoyl substituiertes Piperidinyl, gegebenenfalls einfach oder zweifach, gleichartig oder verschieden durch Methyl, Methoxycarbonyl, Ethoxycarbonyl oder Methylcarbamoyl substituiertes Morpholinyl oder gegebenenfalls einfach oder zweifach, gleichartig oder verschieden durch Methyl, Methoxycarbonyl, Ethoxycarbonyl oder Methylcarbamoyl substituiertes Piperazinyl, oder

R für einen Rest der Formel

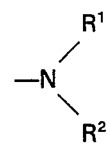

steht, worin

$R^1$ für Wasserstoff, Methyl, Ethyl, n- oder i-Propyl oder Cyclohexyl steht,

$R^2$ für Wasserstoff, Methyl, Ethyl, n- oder i-Propyl, i-Butyl, n-Butyl, n-Hexyl oder n-Dodecyl, Methoxymethyl, Methoxyethyl, Methoxypropyl, Ethoxymethyl, Ethoxyethyl, Allyl, Cyclohexyl, Cyclopentylmethyl, Cyclohexylmethyl oder für gegebenenfalls einfach oder zweifach, gleich oder verschieden durch Fluor, Chlor, Brom, Methyl, Methoxy, Trifluormethyl, Trifluormethoxy und/oder Trifluormethylthio substituiertes Phenyl steht oder

$R^2$ für Benzyl oder Phenethyl steht, wobei jeder dieser Reste im Phenylteil einfach oder zweifach, gleich oder verschieden durch Fluor, Chlor, Brom, Methyl, Methoxy, Trifluormethyl, Trifluormethoxy und/oder Trifluormethylthio substituiert sein kann, oder

$R^1$ und $R^2$ gemeinsam mit dem Stickstoffatom, an das sie gebunden sind, für gegebenenfalls einfach bis dreifach durch Methyl substituiertes Pyrrolidinyl, gegebenenfalls einfach bis dreifach durch Methyl substituiertes Piperidinyl, gegebenenfalls einfach bis dreifach durch Methyl substituiertes Morpholinyl oder für gegebenenfalls am Stickstoff durch Methyl, Ethyl, Acetyl oder Propionyl substituiertes 1,4-Piperazinyl stehen.

Bevorzugte erfindungsgemäße Verbindungen sind auch Additionsprodukte aus Säuren und denjenigen substituierten Azolylmethylcarbinolen der Formel (I), in denen die Substituenten Ar, R und Z die Bedeutungen haben, die bereits vorzugsweise für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der

Elemente und denjenigen substituierten Azolylmethylcarbinolen der Formel (I), in denen die Substituenten Ar, R und Z die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenverträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Im einzelnen seien außer den bei den Herstellungsbeispielen genannten Verbindungen die folgenden substituierten Azolylmethylcarbinole der allgemeinen Formel (I) genannt:

Tabelle 1:

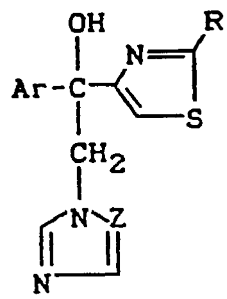

(I)

| Ar | R | Z |
|---|---|---|
| 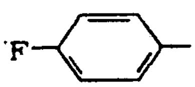 | $-NH-C_2H_5$ | N |
| 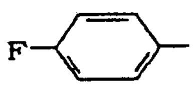 | $-NH-CH_2-CH_2-$ 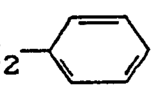 | N |
| 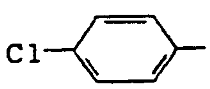 | $-(CH_2)_3-N(CH_3)_2$ | N |
| 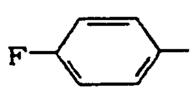 | 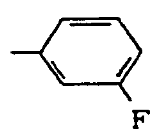 | N |
| 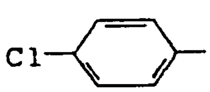 | 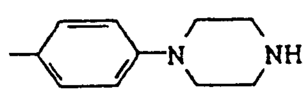 | N |
| 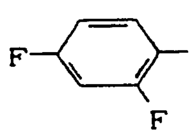 | $-(CH_2)_2-CH_3$ | N |

Tabelle 1 (Fortsetzung)

| Ar | R | Z |
|---|---|---|
| 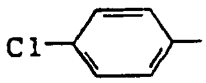 | 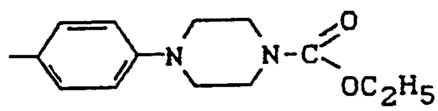 | N |
| 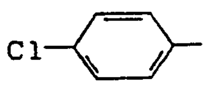 | 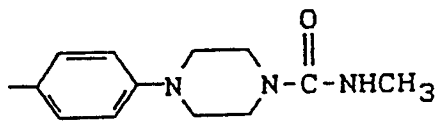 | N |
| 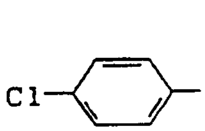 | 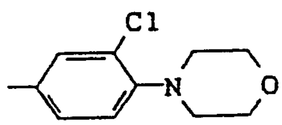 | N |
| 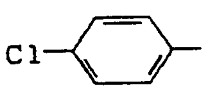 | 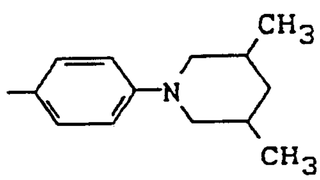 | N |
| 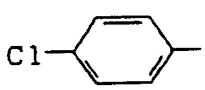 | 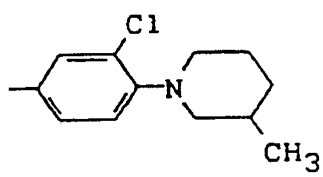 | N |
| 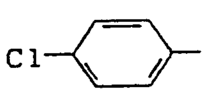 | 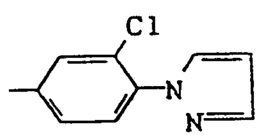 | N |
| 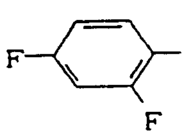 | $-(CH_2)_3-CH_3$ | N |

Verwendet man beispielsweise 1-(4-Chlorphenyl)-1-(2-methylthiazol-4-yl)-2-bromethanol und 1,2,4-Triazol als Ausgangsstoffe, so läßt sich der Reaktionsablauf des erfindungsgemäßen Verfahrens durch das folgende Formelschema darstellen:

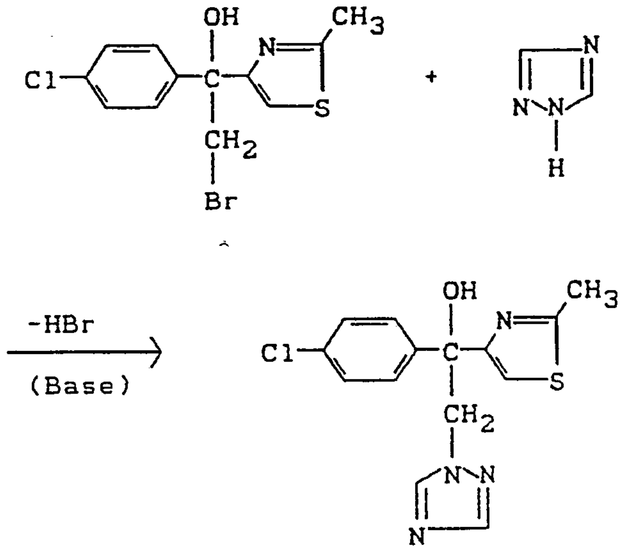


Die zur Durchführung des erfindungsgemäßen Verfahrens als Ausgangsstoffe benötigten substituierten 2-Bromethanole sind durch die Formel (II) allgemein definiert. In dieser Formel (II) stehen Ar und R vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten 2-Bromethanole der Formel (II) sind noch nicht bekannt. Man erhält sie, wenn man Dibromacyloine der Formel

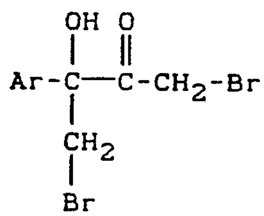
(IV)

in welcher
Ar die oben angegebene Bedeutung hat,
mit Thiocarbonsäureamiden der Formel

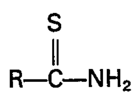
(V)

in welcher
R die oben angegebene Bedeutung hat,
zunächst in Gegenwart einer Base, wie Natriumhydrogencarbonat, sowie in Gegenwart eines Verdünnungsmittels, wie Ethanol, umsetzt und anschließend in Gegenwart einer Säure, wie p-Toluolsulfonsäure, sowie in Gegenwart eines Verdünnungsmittels, wie Dichlormethan, bei Temperaturen zwischen 0°C und 60°C umsetzt (vgl. auch die Herstellungsbeispiele).

Dibromacyloine der Formel (IV) sind bekannt (vgl. Helvetica Chim. Acta *29*, 95—101 [1946]). Sie lassen sich herstellen, wenn man Dibromdiacetyl (vgl. Liebigs Ann. Chem. *249*, 207 [1888]) mit Aromaten der Formel

Ar—H (VI)

in welcher
Ar die oben angegebene Bedeutung hat,
in üblicher Art und Weise durch Friedel-Crafts-Reaktion in Gegenwart eines Katalysators, wie Aluminium-III-chlorid, bei Temperaturen zwischen 0°C und 80°C umsetzt (vgl. auch die Herstellungsbeispiele).

Thiocarbonsäureamide der Formel (V) und Aromaten der Formel (VI) sind allgemein bekannte Verbindungen der organischen Chemie.

Die zur Durchführung des erfindungsgemäßen Verfahrens weiterhin als Ausgangsstoffe benötigten Azole sind durch die Formel (III) allgemein definiert. In dieser Formel (III) steht Z vorzugsweise für diejenigen Reste, die bereits im Zusammenhang mit der Beschreibung der erfindungsgemäßen Stoffe der Formel (I) als bevorzugt für diesen Substituenten genannt wurden.

Die Azole der Formel (III) sind ebenfalls allgemein bekannte Verbindungen der organischen Chemie.

Als Verdünnungsmittel zur Durchführung des erfindungsgemäßen Verfahrens kommen inerte

organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan, Cyclohexan, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, oder Sulfoxide, wie Dimethylsulfoxid, oder Alkohole, wie Methanol, Ethanol, Propanol oder Butanol.

Das erfindungsgemäße Verfahren wird vorzugsweise in Gegenwart eines geeigneten Reaktionshilfsmittels durchgeführt. Als solche kommen alle üblicherweise verwendbaren anorganischen und organischen Basen in Frage, Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat, oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Das erfindungsgemäße Verfahren kann gegebenenfalls auch in einem Zweiphasensystem, wie beispielsweise Wasser/Toluol gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributylmethylphosphoniumbromid, Trimethyl-$C_{13}$/$C_{15}$-alkylammoniumchlorid, Dibenzyl-dimethyl-ammoniummethylsulfat, Dimethyl-$C_{12}$/$C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Die Reaktionstemperaturen können bei der Durchführung des erfindungsgemäßen Verfahrens in einem größeren Bereich variiert werden. Im allgemeinen arbeitet man bei Temperaturen zwischen 20°C und 200°C, vorzugsweise bei Temperaturen zwischen 40°C und 140°C.

Zur Durchführung des erfindungsgemäßen Verfahrens setzt man pro Mol an substituiertem 2-Bromethanol der Formel (II) im allgemeinen 1,0 bis 2,0 Mol, vorzugsweise 1,0 bis 1,5 Mol an Azol der Formel (III) und 1,0 bis 2,0 Mol, vorzugsweise 1,0 bis 1,5 Mol an Reaktionshilfsmittel ein.

Die Reaktionsdurchführung, Aufarbeitung und Isolierung der Reaktionsprodukte erfolgt nach allgemein üblichen Methoden.

Die nach dem erfindungsgemäßen Verfahren erhältlichen Verbindungen der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol, und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide zur Bekämpfung von unerwünschten Mikroorganismen eingesetzt werden.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubensis;

Plasmopara-Arten, wie beispielsweise Plasmopara viticola;

Peronospora-Arten, wie beispielsweise Peronospora pisi oder P. brassicae;

Erysiphe-Arten, wie beispielsweise Erysiphe graminis;

Sphaerotheca-Arten, wie beispielsweise Sphaerotheca fuliginea;

Podosphaera-Arten, wie beispielsweise Podosphaera leucotricha;

Venturia-Arten, wie beispielsweise Venturia inaequalis;

Pyrenophora-Arten, wie beispielsweise Pyrenophora teres oder P. graminea (Konidienform: Drechslera, Syn: Helminthosporium);

Cochliobolus-Arten, wie beispielsweise Cochliobolus sativus (Konidienform: Drechslera, Syn: Helminthosporium);

Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen der Erreger der Halmbruchkrankheit bei Getreide (Pseudocercosporella herpotrichoides) oder gegen den Erreger der Blattfleckenkrankheit der Gerste (Pyrenophora teres) oder gegen den Erreger der Blattfleckenkrankheit des Weizens (Cochliobolus sativus) oder gegen den Erreger der Braunfleckigkeit des Weizens (Lepthosphaeria nodorum), gegen Mehltauarten sowie zur Bekämpfung von Krankheiten im Obst- und Gemüseanbau, wie beispielsweise gegen den Erreger des echten Gurkenmehltaus (Sphaerotheca fuliginea) oder gegen den Erreger des Apfelschorfes (Venturia inaequalis), gegen Cercospora- und Uromyces-Arten wie beispielsweise gegen den Erreger des Bohnenrostes (Uromyces appendiculatus) sowie zur Bekämpfung von Reiskrankheiten, wie beispielsweise gegen den Erreger der Reisfleckenkrankheit (Pyricularia oryzae) einsetzen.

Im Materialschutz lassen sich die erfindungsgemäßen Wirkstoffe zum Schutz von technischen Materialien einsetzen. Unter technischen Materialien sind in diesem Zusammenhang nicht lebende Materialien zu verstehen, die für die Verwendung in der Technik zubereitet worden sind. Beispielsweise können technische Materialien, die durch die erfindungsgemäßen Wirkstoffe vor mikrobieller Veränderung oder Zerstörung geschützt werden sollen, Klebstoffe, Leime, Papier, Karton, Textilien, Leder, Holz, Anstrichmittel, Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), sowie gegen Schleimorganismen und Algen.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole,

Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen wie Fungiziden, Insektiziden, Akariziden und Herbiziden sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001%. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02%, am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele

Beispiel 1

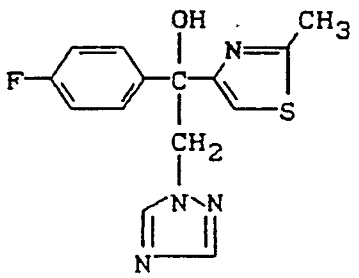

Zu einer Suspension aus 1,8 g (0,06 Mol) Natriumhydrid in 20 ml Dimethylformamid gibt man bei 10°C

tropfenweise unter Rühren eine Lösung von 4,1 g (0,06 Mol) 1,2,4-Triazol in 30 ml Dimethylformamid und nach einer Stunde bei 0°C ebenfalls tropfenweise unter Rühren eine Lösung von 12,6 g (0,04 Mol) 2-Brom-1-(4-fluorphenyl)-1-(2-methylthiazol-4-yl)-ethanol in 30 ml Dimethylformamid. Nach beendeter Zugabe rührt man eine Stunde bei 20°C, zwei weitere Stunden bei 70°C, zwei Stunden bei 90°C und eine weitere Stunde bei 110°C. Zur Aufarbeitung kühlt man die Reaktionsmischung ab, engt im Vakuum ein, nimmt den Rückstand in 300 ml Dichlormethan auf, wäscht zweimal mit jeweils 200 ml Wasser, trocknet über Natriumsulfat, engt im Vakuum ein, chromatographiert über Kieselgel (Laufmittel: Dichlormethan/Methanol 10:1) und kristallisiert das so erhältliche Produkt durch Verrühren mit 150 ml Diisopropylether.

Man erhält 3,5 g (29% der Theorie) an 1-(4-Fluorphenyl)-1-(2-methylthiazol-4-yl)-2-(1,2,4-triazol-1-yl)-ethanol vom Schmelzpunkt 120°C.

Herstellung der Ausgangsverbindung

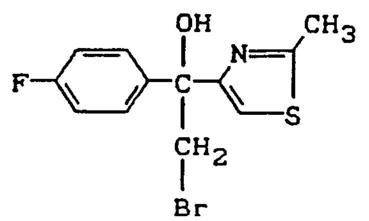

(II-1)

Zu 11,3 g (0.15 Mol) Thioacetamid in 100 ml Ethanol tropft man unter Rühren eine Lösung von 51 g (0,15 Mol) 1,4-Dibrom-3-(4-fluorphenyl)-3-hydroxy-butan-2-on in 200 ml Ethanol, wobei die Temperatur der Reaktionsmischung auf 35°C steigt. Anschließend gibt man 12,6 g (0,15 Mol) Natriumhydrogencarbonat hinzu, rührt 18 Stunden bei Raumtemperatur, engt dann im Vakuum ein, nimmt den Rückstand in 600 ml Dichlormethan auf, wäscht zweimal mit jeweils 200 ml Wasser, versetzt die Mischung mit 5,7 g (0,03 Mol) p-Toluolsulfonsäure und erhitzt für 24 Stunden über einem Wasserabscheider auf Rückflußtemperatur. Zur Aufarbeitung wäscht man das Reaktionsgemisch zweimal mit jeweils 200 ml Wasser, trocknet die organische Phase über Natriumsulfat, engt im Vakuum ein und chromatographiert den Rückstand über Kieselgel (Laufmittel: Toluol).

Man erhält 28,5 g (60% der Theorie) an 2-Brom-1-(4-fluorphenyl)-1-(2-methylthiazol-4-yl)-ethanol als Öl vom Brechungsindex $n_D^{20}$ 1,5812.

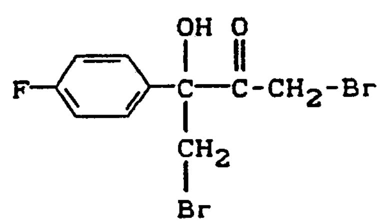

(IV-1)

Zu 133 g (1 Mol) Aluminium-III-chlorid in 1 l Fluorbenzol gibt man innerhalb von 2 Stunden portionsweise 122 g (0,5 Mol) 1,4-Dibrombutan-2,3-dion, wobei die Temperatur der Reaktionsmischung auf 30°C ansteigt. Nach beendeter Zugabe erwärmt man langsam auf 70°C, rührt 2 Stunden bei dieser Temperatur, kühlt ab und gibt den Reaktionsansatz bei 0°C in 3 1 0,2N Salzsäure. Zur Aufarbeitung wird die wässrige Phase abgetrennt und mit 300 ml Toluol extrahiert. Die vereinigten organischen Phasen werden dreimal mit jeweils 500 ml Wasser gewaschen, über Natriumsulfat getrocknet und im Vakuum eingeengt. Der Rückstand wird aus 75 ml Diisopropylether kristallisiert.

Man erhält 87,5 g (52% der Theorie) an 1,4-Dibrom-3-(4-fluorphenyl)-3-hydroxybutan-2-on vom Schmelzpunkt 90°C.

In entsprechender Weise und gemäß den allgemeinen Angaben zur Herstellung erhält man die in der folgenden Tabelle aufgeführten substituierten Azolylmethylcarbinole der Formel (I).

Tabelle 2:

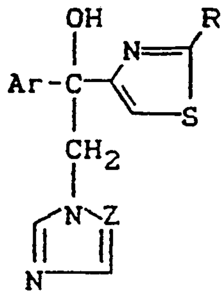

(I)

| Bsp. Nr. | Ar | R | Z | Schmelzpunkt [°C] |
|---|---|---|---|---|
| 2 | 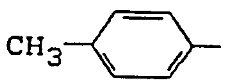 | 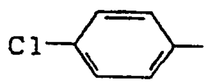 | N | 208 |
| 3 | 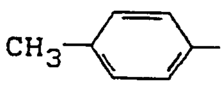 | 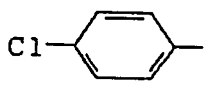 | CH | 228 |
| 4 | 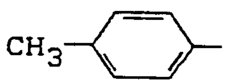 | 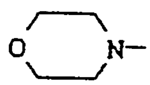 | N | 136 |
| 5 | 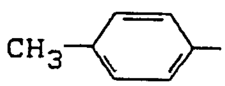 | 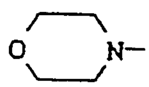 | CH | 156 |
| 6 | 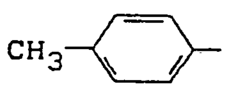 | 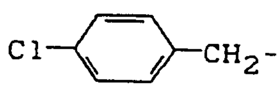 | N | 104 |
| 7 | 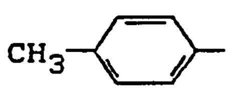 | 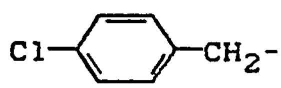 | CH | 122 |
| 8 | 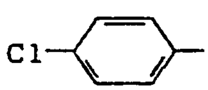 | 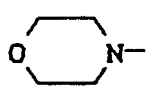 | N | 150 |
| 9 | 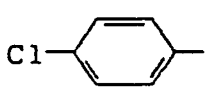 | 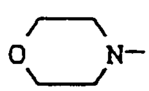 | CH | 188 |

Tabelle 2 (Fortsetzung)

| Bsp. Nr. | Ar | R | Z | Schmelzpunkt [°C] |
|---|---|---|---|---|
| 10 | 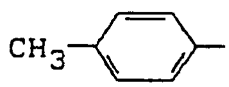 | 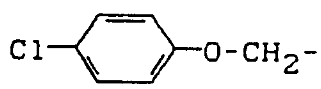 | N | 139 |
| 11 | 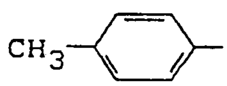 | 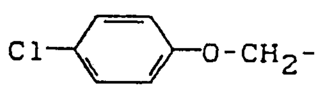 | CH | 138 |
| 12 | 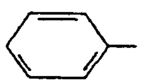 | 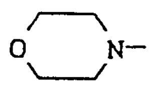 | CH | 160 |
| 13 | 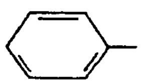 | 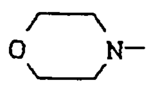 | N | 158 |
| 14 | 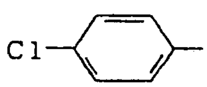 | 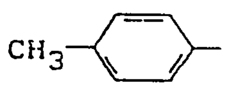 | CH | 208 |
| 15 | 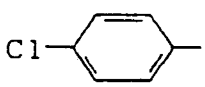 | 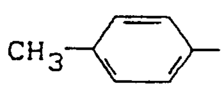 | N | 150 |
| 16 | 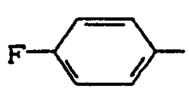 | 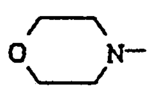 | CH | 142 |
| 17 | 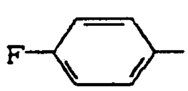 | 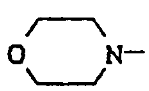 | N | 130 |
| 18 | 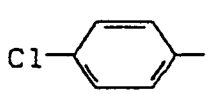 | 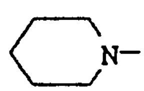 | CH | 174 |
| 19 | 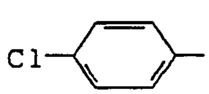 | 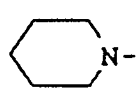 | N | 169 |

Tabelle 2 (Fortsetzung)

| Bsp. Nr. | Ar | R | Z | Schmelzpunkt [°C] |
|---|---|---|---|---|
| 20 | 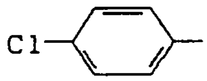 | 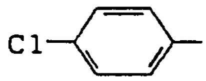 | CH | 230 |
| 21 | 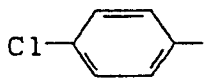 | 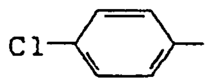 | N | 168 |
| 22 | 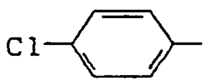 | 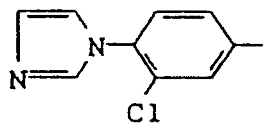 | CH | 200 |
| 23 | 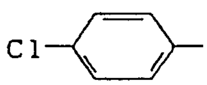 | 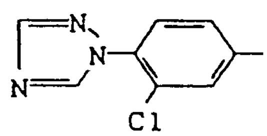 | N | 154 |
| 24 | 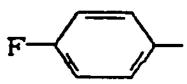 | $CH_3$ | CH | 196 |
| 25 | 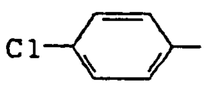 | 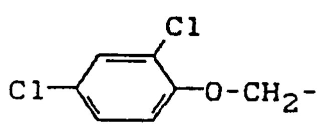 | CH | 136 |
| 26 | 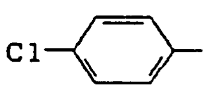 | 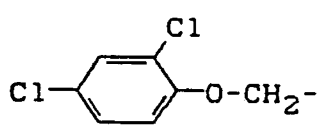 | N | 110 |
| 27 | 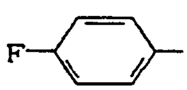 | 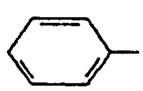 | CH | 149 |
| 28 | 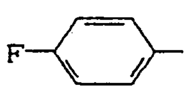 | 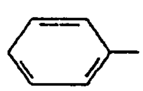 | N | 126 |

Tabelle 2 (Fortsetzung)

| Bsp. Nr. | Ar | R | Z | Schmelzpunkt [°C] |
|---|---|---|---|---|
| 29 | 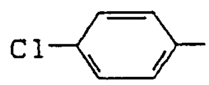 | 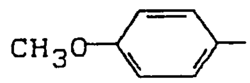 | CH | 205 |
| 30 | 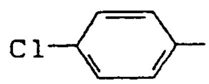 | 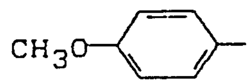 | N | 158 |
| 31 | 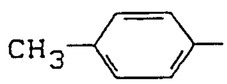 | 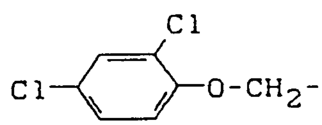 | N | 115 |
| 32 | 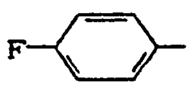 | $(CH_3)_2N$- | CH | 148 |
| 33 | 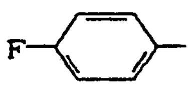 | $(CH_3)_2N$- | N | 130 |
| 34 | 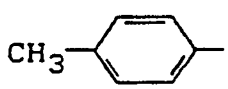 | 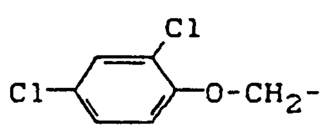 | CH | 96 |
| 35 | 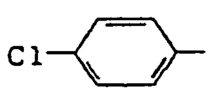 | 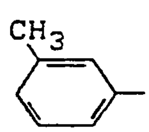 | CH | 182 |
| 36 | 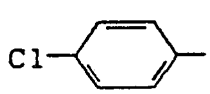 | 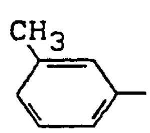 | N | 123 |
| 37 | 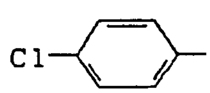 | $CH_3$-NH- | N | 148 |

Tabelle 2 (Fortsetzung)

| Bsp. Nr. | Ar | R | Z | Schmelzpunkt [°C] |
|---|---|---|---|---|
| 38 | 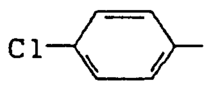 | $CH_3$-NH- | CH | 176 |
| 39 | 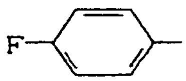 | 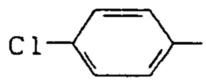 | CH | 225 |
| 40 | 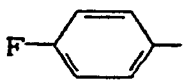 | 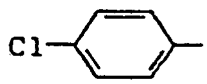 | N | 145 |
| 41 | 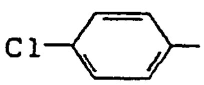 | 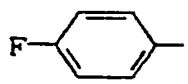 | N | 130 |
| 42 | 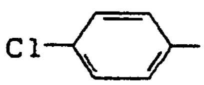 | 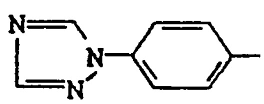 | N | 164 |
| 43 | 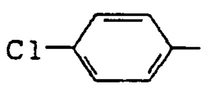 | 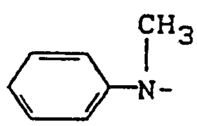 | N | 115 |
| 44 | 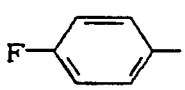 | 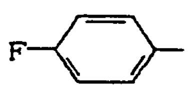 | N | 110 |
| 45 | 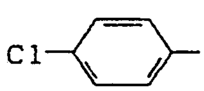 | 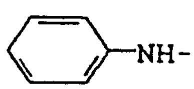 | N | 185 |
| 46 | 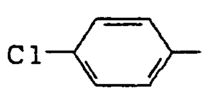 | $CH_2$=CH-$CH_2$-NH- | N | 126 |
| 47 | 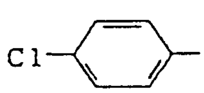 | 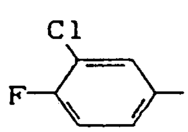 | N | 140 |

Tabelle 2 (Fortsetzung)

| Bsp. Nr. | Ar | R | Z | Schmelzpunkt [°C] |
|---|---|---|---|---|
| 48 | 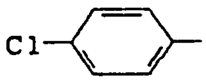 | 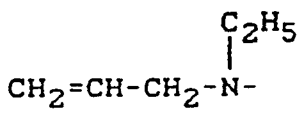 | N | 80 |
| 49 | 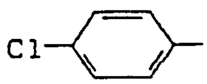 | $(C_2H_5)_2N-$ | N | 116 |
| 50 | 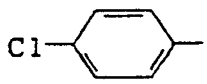 | 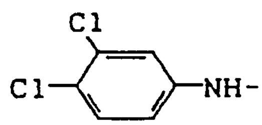 | N | 140 |
| 51 | 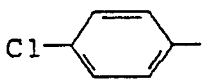 | $C_2H_5-NH-$ | N | 124 |
| 52 | 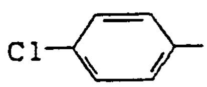 | 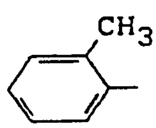 | N | 110 |
| 53 | 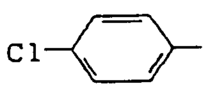 | 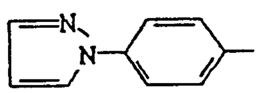 | N | 170 |
| 54 | 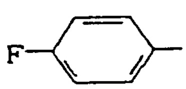 | 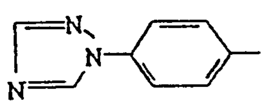 | N | 152 |
| 55 | 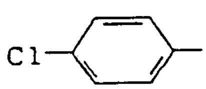 | 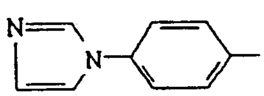 | N | 146 |
| 56 | 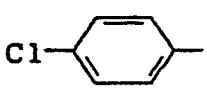 | 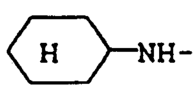 | N | 175 |
| 57 | 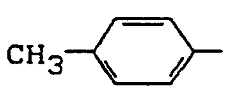 | $CH_3$ | N | 150 |

Tabelle 2 (Fortsetzung)

| Bsp. Nr. | Ar | R | Z | Schmelzpunkt [°C] |
|---|---|---|---|---|
| 58 | 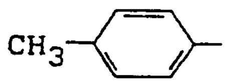 | 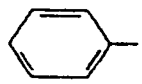 | N | 142 |
| 59 | 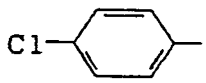 | 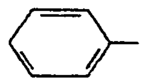 | N | 150 |
| 60 | 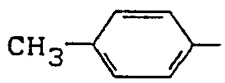 | $(CH_3)_2N-$ | N | 142 |
| 61 | 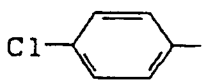 | $(CH_3)_2N-$ | N | 130 |
| 62 | 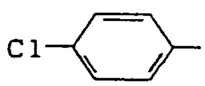 | 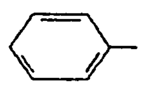 | CH | 171 |
| 63 | 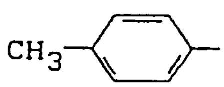 | $(CH_3)_2N-$ | CH | 163 |
| 64 | 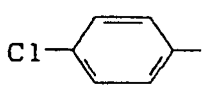 | $(CH_3)_2N-$ | CH | 177 |
| 65 | 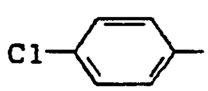 | $CH_3$ | N | 106 |
| 66 | 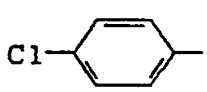 | $CH_3$ | CH | 182 |
| 67 | 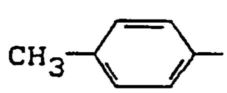 | $CH_3$ | CH | 160 |

Tabelle 2 (Fortsetzung)

| Bsp. Nr. | Ar | R | Z | Schmelzpunkt [°C] |
|---|---|---|---|---|
| 68 | 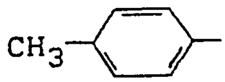 | 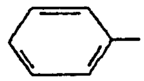 | CH | 165 |
| 69 | 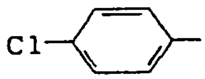 | 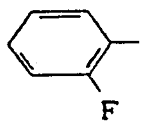 | N | 112 |
| 70 | 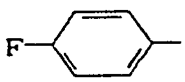 | 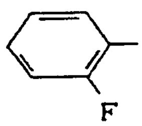 | N | 110 |
| 71 | 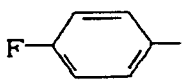 | 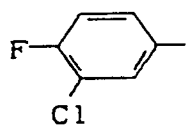 | N | 128 |
| 72 | 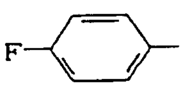 | 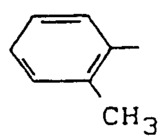 | N | 111 |
| 73 | 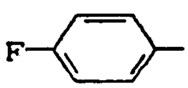 | 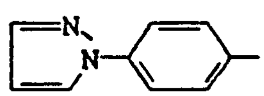 | N | 200 |
| 74 | 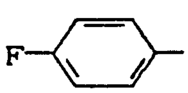 | 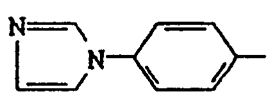 | N | 162 |
| 75 | 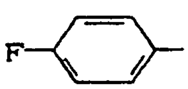 | 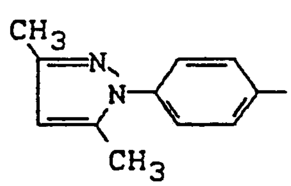 | N | 158 |
| 76 | 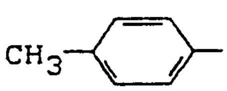 | 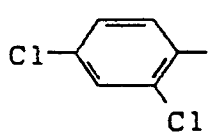 | N | 175 |

Tabelle 2 (Fortsetzung)

| Bsp. Nr. | Ar | R | Z | Schmelzpunkt [° C] |
|---|---|---|---|---|
| 77 | 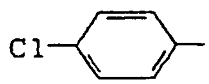 | 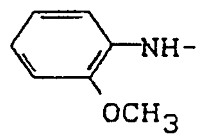 | N | 186 |
| 78 | 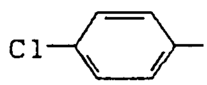 | $C_2H_5$ | N | 100 |
| 79 | 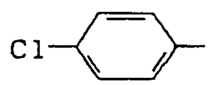 | $CH_3-(CH_2)_3-NH-$ | N | 114 |
| 80 | 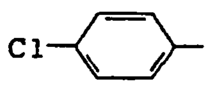 | $CH_3-(CH_2)_2-NH-$ | N | 118 |
| 81 | 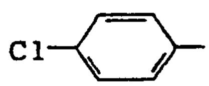 | $(CH_3)_2CH-CH_2-NH-$ | N | 140 |
| 82 | 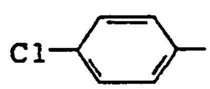 | 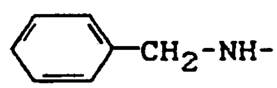 | N | 162 |
| 83 | 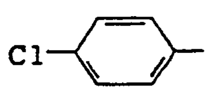 | 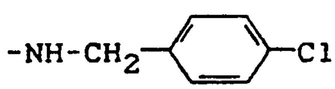 | N | 138 |
| 84 | 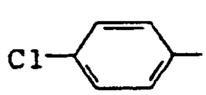 | 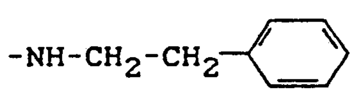 | N | 108 |
| 85 | 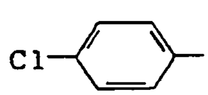 | $-NH-(CH_2)_5-CH_3$ | N | 84 |
| 86 | 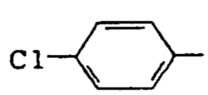 | 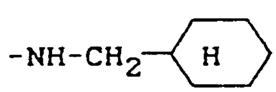 | N | 98 |

Tabelle 2 (Fortsetzung)

| Bsp. Nr. | Ar | R | Z | Schmelzpunkt [°C] |
|---|---|---|---|---|
| 87 | 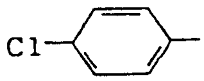 | $-NH-(CH_2)_{11}-CH_3$ | N | 88 |
| 88 | 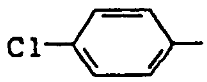 | $-NH-CH_2-CH_2-OCH_3$ | N | 106 |
| 89 | 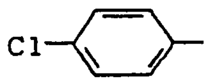 | $-NH-(CH_2)_3-OCH_3$ | N | 68 |
| 90 | 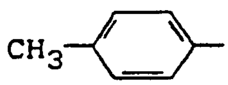 | 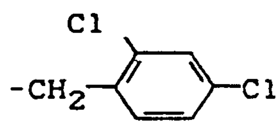 | N | 90 |
| 91 | 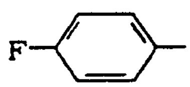 | 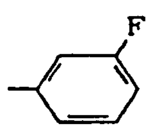 | N | 130 |
| 92 | 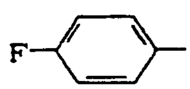 | 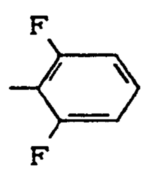 | N | 118 |
| 93 | 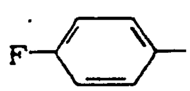 | 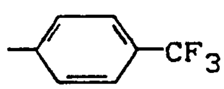 | N | 146 |
| 94 | 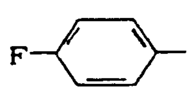 | 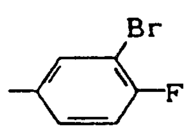 | N | 126 |
| 95 | 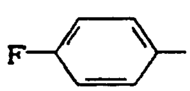 | 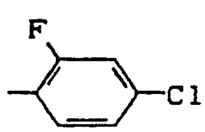 | N | 144 |

Tabelle 2 (Fortsetzung)

| Bsp. Nr. | Ar | R | Z | Schmelzpunkt [°C] bzw. Brechungsindex |
|---|---|---|---|---|
| 96 | 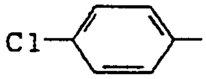 | $-CH(CH_3)_2$ | N | 140 |
| 97 | 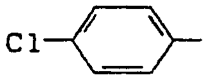 | $-C(CH_3)_3)$ | N | 132 |
| 98 | 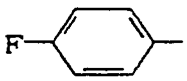 | 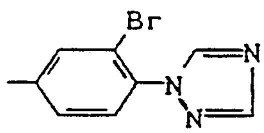 | N | 134 |
| 99 | 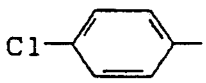 | $-(CH_2)_3-CH_3$ | N | $n_D^{20}=1,5739$ |
| 100 | 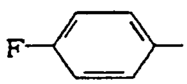 | 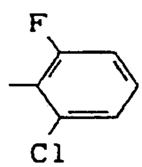 | N | $n_D^{20}=1,5933$ |
| 101 | 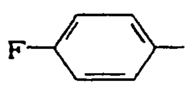 | 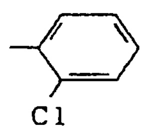 | N | 102 |

Anwendungsbeispiele

In den folgenden Anwendungsbeispielen wurden die nachstehend aufgeführten Verbindungen als Vergleichssubstanzen eingesetzt:

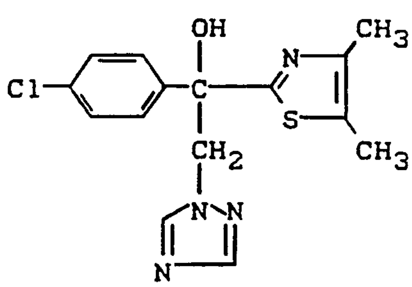

(A)

1-(4-Chlorphenyl)-1-(4,5-dimethylthiazol-2-yl)-2-(1,2,4-triazol-1-yl)-ethanol

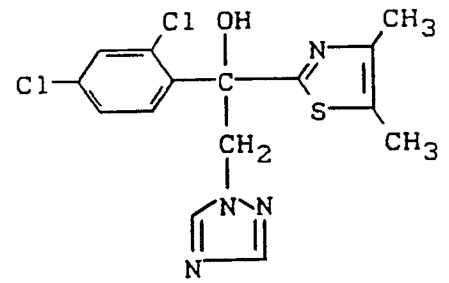

(B)

1-(2,4-Dichlorphenyl)-1-(4,5-dimethylthiazol-2-yl)-2-(1,2,4-triazol-1-yl)-ethanol (beide bekannt aus DE—A 3 413 173 und EP—A 0 158 205)

Beispiel A

Leptosphaeria nodorum-Test (Weizen)/protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100% relativer Luftfeuchtigkeit in einer Inkubationskabine.

Die Pflanzen werden in einem Gewächshaus bei einer Temperatur von ca. 15°C und einer relativen Luftfeuchtigkeit von ca. 80% aufgestellt.

10 Tage nach der Inokulation erfolgt die Auswertung.

In diesem Test zeigen die in den Beispielen 1, 6, 7, 9, 10, 15, 17, 23, 28, 31, 32, 36, 37, 59, 60, 61, 62 und 68 aufgeführten erfindungsgemäßen Stoffe eine wesentlich bessere Wirksamkeit als die Vergleichssubstanz (A).

Beispiel B

Uromyces-Test (Buschbohne)/protektiv

Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Uredosporensuspension des Bohnenrosterregers (Uromyces appendiculatus) inokuliert und verbleiben 1 Tag in einer dunklen Feuchtkammer bei 20 bis 22°C und 100% relativer Luftfeuchtigkeit.

Die Pflanzen werden dann unter intensiver Belichtung für 9 Tage bei 20 bis 22°C und einer relativen Luftfeuchtigkeit von 70 bis 80% im Gewächshaus aufgestellt.

10 Tage nach der Inokulation erfolgt die Auswertung.

In diesem Test zeigen die in den Beispielen 23, 25, 27, 28 und 40 aufgeführten erfindungsgemäßen Wirkstoffe eine wesentlich bessere Wirksamkeit als die Vergleichssubstanz (B).

Beispiel C

Wachstum bei Baumwolle

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des fünften Folgeblattes angezogen. In diesem Stadium werden die Pflanzen tropfnaß mit den Wirkstoffzubereitungen besprüht. Nach zwei Wochen wird bei allen Pflanzen der Zuwachs gemessen und das Wachstum in Prozent des Zuwachses der Kontrollpflanzen berechnet. Es bedeuten 100% Wachstum ein Wachstum entsprechend dem der Kontrollpflanzen und 0% den Stillstand des Wachstums. Werte über 100% kennzeichnen eine Wuchsförderung.

In diesem Test zeigt die im Beispiel 28 aufgeführte erfindungsgemäße Verbindung eine sehr starke wuchshemmende Wirkung.

Beispiel D

Wachstum bei Sojabohnen

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach zwei Wochen wird bei allen Pflanzen der Zuwachs gemessen und das Wachstum in Prozent des Zuwachses der Kontrollpflanzen berechnet. Es bedeuten 100% Wachstum ein Wachstum entsprechend dem der Kontrollpflanzen und 0% den Stillstand des Wachstums. Werte über 100% kennzeichnen eine Wuchsförderung.

In diesem Test zeigt die im Beispiel 16 aufgeführte erfindungsgemäße Verbindung eine sehr starke wuchshemmende Wirkung.

**Patentansprüche**

1. Substituierte Azolylmethylcarbinole der Formel

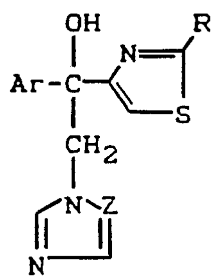

(I)

in welcher

Ar für Phenyl steht, welches einfach oder mehrfach, gleichartig oder verschieden substituiert sein kann durch Halogen, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Halogenalkoxy mit 1 bis 4 Kohlenstoffatomen und 1 bis 9 gleichen oder verschiedenen Halogenatomen und/oder durch geradkettiges oder verzweigtes Halogenalkylthio mit 1 bis 4 Kohlenstoffatomen und 1 bis 9 gleichen oder verschiedenen Halogenatomen,

Z für Stickstoff oder eine CH-Gruppe steht und

R für geradkettiges oder verzweigtes Alkyl mit 1 bis 6 Kohlenstoffatomen, geradkettiges oder verzweigtes Dialkylaminoalkyl mit 1 bis 4 Kohlenstoffatomen in jeder Alkylgruppe, geradkettiges oder verzweigtes Alkenyl mit 3 bis 6 Kohlenstoffatomen, geradkettiges oder verzweigtes Alkinyl mit 3 bis 6 Kohlenstoffatomen, geradkettiges oder verzweigtes Alkoxyalkyl mit 1 bis 6 Kohlenstoffatomen im Alkylteil und 1 bis 6 Kohlenstoffatomen im Alkoxyteil steht, oder

R für Aralkyl mit 1 bis 4 Kohlenstoffatomen im Alkylteil und 6 bis 10 Kohlenstoffatomen im Arylteil steht, wobei der Arylteil einfach oder mehrfach, gleichartig oder verschieden substituiert sein kann durch Halogen, Cyano, Nitro, Phenyl, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Halogenalkoxy mit 1 bis 4 Kohlenstoffatomen und 1 bis 9 gleichen oder verschiedenen Halogenatomen und/oder durch geradkettiges oder verzweigtes Halogenalkylthio mit 1 bis 4 Kohlenstoffatomen und 1 bis 9 gleichen oder verschiedenen Halogenatomen, oder durch einen über N verknüpften fünf- oder sechsgliedrigen Stickstoffheterocyclus, der gegebenenfalls weitere Heteroatome enthalten kann und einfach oder mehrfach, gleichartig oder verschieden substituiert sein kann durch geradkettiges oder verzweigtes Alkyl mit 1 bis 4 Kohlenstoffatomen, geradkettiges oder verzweigtes Alkoxycarbonyl mit 1 bis 4 Kohlenstoffatomen im Alkoxyteil und/oder durch Alkylcarbamoyl mit 1 bis 4 Kohlenstoffatomen im Alkylteil, oder

R für Aryloxyalkyl mit 1 bis 4 Kohlenstoffatomen im Alkylteil und 6 bis 10 Kohlenstoffatomen im Arylteil steht, wobei der Arylteil einfach oder mehrfach, gleichartig oder verschieden substituiert sein kann

durch Halogen, Cyano, Nitro, Phenyl, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Halogenalkoxy mit 1 bis 4 Kohlenstoffatomen und 1 bis 9 gleichen oder verschiedenen Halogenatomen und/oder durch geradkettiges oder verzweigtes Halogenalkylthio mit 1 bis 4 Kohlenstoffatomen und 1 bis 9 gleichen oder verschiedenen Halogenatomen, oder durch einen über N verknüpften fünf- oder sechsgliedrigen Stickstoffheterocyclus, der gegebenenfalls weitere Heteroatome enthalten kann und einfach oder mehrfach, gleichartig oder verschieden substituiert sein kann durch geradkettiges oder verzweigtes Alkyl mit 1 bis 4 Kohlenstoffatomen, geradkettiges oder verzweigtes Alkoxycarbonyl mit 1 bis 4 Kohlenstoffatomen im Alkoxyteil und/oder durch Alkylcarbamoyl mit 1 bis 4 Kohlenstoffatomen im Alkylteil, oder

R für Aryl mit 6 bis 10 Kohlenstoffatomen steht, wobei der Aryl-Rest einfach oder mehrfach, gleichartig oder verschieden substituiert sein kann durch Halogen, Cyano, Nitro, Phenyl, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Halogenalkoxy mit 1 bis 4 Kohlenstoffatomen und 1 bis 9 gleichen oder verschiedenen Halogenatomen und/oder durch geradkettiges oder verzweigtes Halogenalkylthio mit 1 bis 4 Kohlenstoffatomen und 1 bis 9 gleichen oder verschiedenen Halogenatomen, durch einen über N verknüpften fünf- oder sechsgliedrigen Stickstoffheterocyclus, der gegebenenfalls weitere Heteroatome enthalten kann und einfach oder mehrfach, gleichartig oder verschieden substituiert sein kann durch geradkettiges oder verzweigtes Alkyl mit 1 bis 4 Kohlenstoffatomen, geradkettiges oder verzweigtes Alkoxycarbonyl mit 1 bis 4 Kohlenstoffatomen im Alkoxyteil und/oder durch Alkylcarbamoyl mit 1 bis 4 Kohlenstoffatomen im Alkylteil, oder

R für einen Rest der Formel

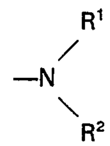

steht, worin

$R^1$ für Wasserstoff, geradkettiges oder verzweigtes Alkyl mit 1 bis 6 Kohlenstoffatomen, geradkettiges oder verzweigtes Alkenyl mit 2 bis 6 Kohlenstoffatomen, geradkettiges oder verzweigtes Alkinyl mit 2 bis 6 Kohlenstoffatomen oder für Cycloalkyl mit 3 bis 7 Kohlenstoffatomen steht und

$R^2$ für Wasserstoff, geradkettiges oder verzweigtes Alkyl mit 1 bis 16 Kohlenstoffatomen, geradkettiges oder verzweigtes Alkoxyalkyl mit 1 bis 4 Kohlenstoffatomen im Alkylteil und 1 bis 4 Kohlenstoffatomen im Alkoxyteil, geradkettiges oder verzweigtes Alkenyl mit 2 bis 6 Kohlenstoffatomen, geradkettiges oder verzweigtes Alkinyl mit 2 bis 6 Kohlenstoffatomen, Cycloalkyl mit 3 bis 7 Kohlenstoffatomen, Cycloalkylalkyl mit 3 bis 7 Kohlenstoffatomen im Cycloalkylteil und 1 bis 4 Kohlenstoffatomen im Alkylteil oder für Phenyl steht, das einfach oder mehrfach, gleichartig oder verschieden substituiert sein kann durch Halogen, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Halogenalkoxy mit 1 bis 4 Kohlenstoffatomen und 1 bis 9 gleichen oder verschiedenen Halogenatomen und/oder durch geradkettiges oder verzweigtes Halogenalkylthio mit 1 bis 4 Kohlenstoffatomen und 1 bis 9 gleichen oder verschiedenen Halogenatomen, oder

$R^2$ für Aralkyl mit 1 bis 4 Kohlenstoffatomen im Alkylteil und 6 bis 10 Kohlenstoffatomen im Arylteil steht, wobei der Arylteil einfach oder mehrfach, gleichartig oder verschieden substituiert sein kann durch Halogen, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, durch geradkettiges oder verzweigtes Halogenalkoxy mit 1 bis 4 Kohlenstoffatomen und 1 bis 9 gleichen oder verschiedenen Halogenatomen, und/oder durch geradkettiges oder verzweigtes Halogenalkylthio mit 1 bis 4 Kohlenstoffatomen und 1 bis 9 gleichen oder verschiedenen Halogenatomen, oder

$R^1$ und $R^2$ gemeinsam mit dem Stickstoffatom, an das sie gebunden sind, für einen gesättigten Heterocyclus mit 5 bis 7 Ringgliedern stehen, wobei der Heterocyclus ein weiteres Heteroatom enthalten kann und wobei der Heterocyclus einfach oder mehrfach, gleichartig oder verschieden substituiert sein kann durch geradkettiges oder verzweigtes Alkyl mit 1 bis 4 Kohlenstoffatomen, geradkettiges oder verzweigtes Hydroxyalkyl mit 1 bis 4 Kohlenstoffatomen, geradkettiges oder verzweigtes Alkanoyl mit 1 bis 4 Kohlenstoffatomen im Alkanteil und/oder durch Alkanoyloxyalkyl mit 1 bis 4 Kohlenstoffatomen im Alkanteil und 1 bis 4 Kohlenstoffatomen im Oxyalkylteil,

sowie deren Säureadditions-Salze und Metallsalz-Komplexe.

2. Verfahren zur Herstellung von substituierten Azolylmethylcarbinolen der Formel

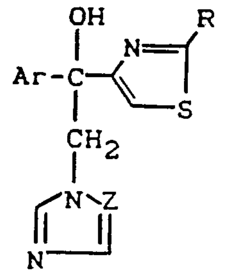

(I)

in welcher

Ar, R und Z die im Anspruch 1 angegebenen Bedeutungen haben,

sowie von deren Säureadditions-Salzen und Metallsalz-Komplexen, dadurch gekennzeichnet, daß man substituierte 2-Brom-ethanole der Formel

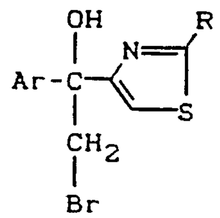

(II)

in welcher

Ar und R die angegebene Bedeutung haben,

mit Azolen der Formel

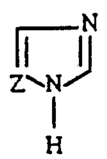

(III)

in welcher

Z die oben angegebene Bedeutung hat,

gegebenenfalls in Gegenwart eines Verdünnungsmittels und gegebenenfalls in Gegenwart eines Reaktionshilfsmittels sowie gegebenenfalls in Gegenwart eines Phasentransferkatalysators umsetzt und gegebenenfalls anschließend eine Säure oder ein Metallsalz addiert.

3. Fungizide und pflanzenwuchsregulierende Mittel, gekennzeichnet durch einen Gehalt an mindestens einem substituierten Azolylmethylcarbinol der Formel (I) gemäß Anspruch 1 bzw. an einem Säureadditions-Salz oder Metallsalz-Komplexe eines substituierten Azolylmethylcarbinols der Formel (I).

4. Verwendung von substituierten Azolylmethylcarbinolen der Formel (I) gemäß Anspruch 1 bzw. von deren Säureadditions-Salzen und Metallsalz-Komplexen zur Bekämpfung von Pilzen sowie zur Regulierung des Pflanzenwachstums.

5. Verfahren zur Bekämpfung von Pilzen sowie zur Regulierung des Pflanzenwachstums, dadurch gekennzeichnet, daß man substituierte Azolylmethylcarbinole der Formel (I) gemäß Anspruch 1 bzw. deren Säureadditions-Salze oder Metallsalz-Komplexe auf die Pflanzen und/oder deren Lebensraum ausbringt.

6. Verfahren zur Herstellung von fungiziden und pflanzenwuchsregulierenden Mitteln, dadurch gekennzeichnet, daß man substituierte Azolylmethylcarbinole der Formel (I) gemäß Anspruch 1 bzw. deren Säureadditions-Salze oder Metallsalz-Komplexe mit Streckmitteln und/oder oberflächenaktiven Stoffen vermischt.

7. Substituierte 2-Brom-ethanole der Formel

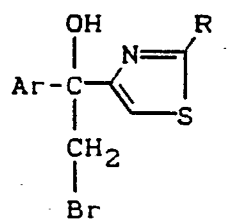

(II)

in welcher

Ar und R die im Anspruch 1 angegebenen Bedeutungen haben.

8. Verfahren zur Herstellung von substituierten 2-Brom-ethanolen der Formel

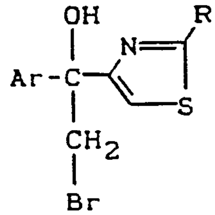

(II)

in welcher

Ar und R die oben angegebenen Bedeutungen haben,

dadurch gekennzeichnet, daß man Dibromacyloine der Formel

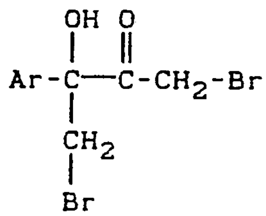

(IV)

in welcher

Ar die oben angegebene Bedeutung hat,

mit Thiocarbonsäureamiden der Formel

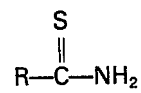

(V)

in welcher

R die oben angegebene Bedeutung hat,

zunachst in Gegenwart einer Base sowie in Gegenwart eines Verdünnungsmittels umsetzt und anschließend in Gegenwart einer Säure sowie in Gegenwart eines Verdünnungsmittels umsetzt.

**Revendications**

1. Azolylméthylcarbinols substitués de formule

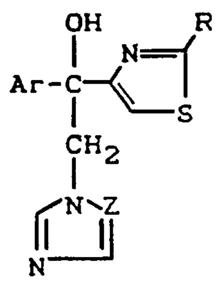

(I)

dans laquelle

Ar représente un groupe phényle qui peut être substitué une ou plusieurs fois, identiquement ou différemment, par un halogène, un groupe alkyle à chaîne droite ou à chaîne ramifiée ayant 1 à 4 atomes de carbone, un groupe alkoxy à chaîne droite ou à chaîne ramifiée ayant 1 à 4 atomes de carbone, un groupe alkylthio à chaîne droite ou à chaîne ramifiée ayant 1 à 4 atomes de carbone, un groupe halogènalkyle à chaîne droite ou à chaîne ramifiée ayant 1 à 4 atomes de carbone et 1 à 9 atomes d'halogènes identiques ou différents, un groupe halogénalkoxy à chaîne droite ou à chaîne ramifiée ayant 1 à 4 atomes de carbone et 1 à 9 atomes d'halogènes identiques ou différents et/ou par un groupe halogénalkylthio à chaîne droite ou à chaîne ramifiée ayant 1 à 4 atomes de carbone et 1 à 9 atomes d'halogènes identiques ou différents,

Z représente l'azote ou un groupe CH et

R est un groupe alkyle à chaîne droite ou à chaîne ramifiée ayant 1 à 6 atomes de carbone, un groupe dialkylaminoalkyle à chaîne droite ou à chaîne ramifiée ayant 1 à 4 atomes de carbone dans chaque groupe alkyle, un groupe alcényle à chaîne droite ou à chaîne ramifiée ayant 3 à 6 atomes de carbone, un groupe alcinyle à chaîne droite ou à chaîne ramifiée ayant 3 à 6 atomes de carbone, un groupe alkoxyalkyle à chaîne droite ou à chaîne ramifiée ayant 1 à 6 atomes de carbone dans la partie alkyle et 1 à 6 atomes de carbone dans la partie alkoxy, ou

R est un groupe aralkyle ayant 1 à 4 atomes de carbone dans la partie alkyle et 6 à 10 atomes de

carbone dans la partie aryle, la partie aryle pouvant être substituée une ou plusieurs fois, identiquement ou différemment, par un halogène, un groupe cyano, nitro, phényle, un groupe alkyle à chaîne droite ou à chaîne ramifiée ayant 1 à 4 atomes de carbone, un groupe alkoxy à chaîne droite ou à chaîne ramifiée ayant 1 à 4 atomes de carbone, un groupe alkylthio à chaîne droite ou à chaîne ramifiée ayant 1 à 4 atomes de carbone, un groupe halogénalkyle à chaîne droite ou à chaîne ramifiée ayant 1 à 4 atomes de carbone et 1 à 9 atomes d'halogènes identiques ou différents, un groupe halogénalkoxy à chaîne droite ou à chaîne ramifiée ayant 1 à 4 atomes de carbone et 1 à 9 atomes d'halogènes identiques ou différents et/ou un groupe halogénalkylthio à chaîne droite ou à chaîne ramifiée ayant 1 à 4 atomes de carbone et 1 à 9 atomes d'halogène identiques ou différents, ou par un hétérocycle azoté pentagonal ou hexagonal lié par l'intermédiaire de N, qui peut contenir le cas échéant d'autres hétéroatomes et qui peut être substitué une ou plusieurs fois, identiquement ou différemment, par un radical alkyle à chaîne droite ou à chaîne ramifiée ayant 1 à 4 atomes de carbone, un radical alkoxycarbonyle à chaîne droite ou à chaîne ramifiée ayant 1 à 4 atomes de carbone dans la partie alkoxy et/ou un radical alkylcarbamoyle ayant 1 à 4 atomes de carbone dans la partie alkyle, ou bien

R est un groupe aryloxyalkyle ayant 1 à 4 atomes de carbone dans la partie alkyle et 6 à 10 atomes de carbone dans la partie aryle, la partie aryle pouvant être substituée une ou plusieurs fois, identiquement ou différemment, par un halogène, un radical cyano, nitro, phényle, alkyle à chaîne droite ou à chaîne ramifiée ayant 1 à 4 atomes de carbone, alkoxy à chaîne droite ou à chaîne ramifiée ayant 1 à 4 atomes de carbone, alkylthio à chaîne droite ou à chaîne ramifiée ayant 1 à 4 atomes de carbone, halogénalkyle à chaîne droite ou à chaîne ramifiée ayant 1 à 4 atomes de carbone et 1 à 9 atomes d'halogènes identiques ou différents, un radical halogénalkoxy à chaîne droite ou à chaîne ramifiée ayant 1 à 4 atomes de carbone et 1 à 9 atomes d'halogènes identiques ou différents et/ou par un radical halogénalkylthio à chaîne droite ou à chaîne ramifiée ayant 1 à 4 atomes de carbone et 1 à 9 atomes d'halogène identiques ou différents, ou par un hétérocycle azoté pentagonal ou hexagonal lié par l'intermédiaire de N, qui peut comporter le cas échéant d'autres hétéroatomes et qui peut être substitué une ou plusieurs fois, identiquement ou différemment, par un radical alkyle à chaîne droite ou à chaîne ramifiée ayant 1 à 4 atomes de carbone, un radical alkoxycarbonyle à chaîne droite ou à chaîne ramifiée ayant 1 à 4 atomes de carbone dans la partie alkoxy et/ou par un radical alkylcarbamoyle ayant 1 à 4 atomes de carbone dans la partie alkyle, ou bien

R est un reste aryle ayant 6 à 10 atomes de carbone, le reste aryle pouvant être substitué une ou plusieurs fois identiquement ou différemment par un halogène, un radical cyano, nitro, phényle, alkyle à chaîne droite ou à chaîne ramifiée ayant 1 à 4 atomes de carbone, alkoxy à chaîne droite ou à chaîne ramifiée ayant 1 à 4 atomes de carbone, alkylthio à chaîne droite ou à chaîne ramifiée ayant 1 à 4 atomes de carbone, halogénalkyle à chaîne droite ou à chaîne ramifiée ayant 1 à 4 atomes de carbone et 1 à 9 atomes d'halogènes identiques ou différents, halogénalkoxy à chaîne droite ou à chaîne ramifiée ayant 1 à 4 atomes de carbone et 1 à 9 atomes d'halogènes identiques ou différents et/ou par un radical halogénalkylthio à chaîne droite ou à chaîne ramifiée ayant 1 à 4 atomes de carbone et 1 à 9 atomes d'halogènes identiques ou différents, par un hétérocycle azoté pentagonal ou hexagonal lié par l'intermédiaire de N, qui peut contenir le cas échéant d'autres hétéroatomes et qui peut être substitué une ou plusieurs fois, identiquement ou différemment, par un radical alkyle à chaîne droite ou à chaîne ramifiée ayant 1 à 4 atomes de carbone, un radical alkoxycarbonyle à chaîne droite ou à chaîne ramifiée ayant 1 à 4 atomes de carbone dans la partie alkoxy et/ou par un radical alkylcarbamoyle ayant 1 à 4 atomes de carbone dans la partie alkyle, ou bien

R est un reste de formule

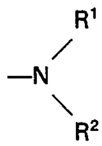

dans laquelle

$R^1$ représente l'hydrogène, un groupe alkyle à chaîne droite ou à chaîne ramifiée ayant 1 à 6 atomes de carbone, un groupe alcényle à chaîne droite ou à chaîne ramifiée ayant 2 à 6 atomes de carbone, un groupe alcinyle à chaîne droite ou à chaîne ramifiée ayant 2 à 6 atomes de carbone ou un groupe cycloalkyle de 3 à 7 atomes de carbone et

$R^3$ représente l'hydrogène, un groupe alkyle à chaîne droite ou à chaîne ramifiée ayant 1 à 16 atomes de carbone, un groupe alkoxyalkyle à chaîne droite ou à chaîne ramifiée ayant 1 à 4 atomes de carbone dans la partie alkyle et 1 à 4 atomes de carbone dans la partie alkoxy, un groupe alcényle à chaîne droite ou à chaîne ramifiée ayant 2 à 6 atomes de carbone, un groupe alcinyle à chaîne droite ou à chaîne ramifiée ayant 2 à 6 atomes de carbone, un groupe cycloalkyle de 3 à 7 atomes de carbone, un groupe cycloalkyl-alkyle de 3 à 7 atomes de carbone dans la partie cycloalkyle et de 1 à 4 atomes de carbone dans la partie alkyle ou un groupe phényle qui peut être substitué une ou plusieurs fois, identiquement ou différemment, par un halogène, un radical alkyle à chaîne droite ou à chaîne ramifiée ayant 1 à 4 atomes de carbone, un radical alkoxy à chaîne droite ou à chaîne ramifiée ayant 1 à 4 atomes de carbone, un radical alkylthio à chaîne droite ou à chaîne ramifiée ayant 1 à 4 atomes de carbone, un radical halogénalkyle à chaîne droite ou à chaîne ramifiée ayant 1 à 4 atomes de carbone et 1 à 9 atomes d'halogènes identiques ou différents, un radical halogénalkoxy à chaîne droite ou à chaîne ramifiée ayant 1 à 4 atomes de carbone et 1 à 9 atomes

d'halogènes identiques ou différents et/ou par un radical halogénalkylthio à chaîne droite ou à chaîne ramifiée ayant 1 à 4 atomes de carbone et 1 à 9 atomes d'halogènes identiques ou différents, ou bien

$R^2$ est un groupe aralkyle ayant 1 à 4 atomes de carbone dans la partie alkyle et 6 à 10 atomes de carbone dans la partie aryle, la partie aryle pouvant être substituée une ou plusieurs fois, identiquement ou différemment, par un halogène, un radical alkyle à chaîne droite ou à chaîne ramifiée ayant 1 à 4 atomes de carbone, un radical alkoxy à chaîne droite ou à chaîne ramifiée ayant 1 à 4 atomes de carbone, un radical alkylthio à chaîne droite ou à chaîne ramifiée ayant 1 à 4 atomes de carbone, un radical halogénalkyle à chaîne droite ou à chaîne ramifiée ayant 1 à 4 atomes de carbone et 1 à 9 atomes d'halogènes identiques ou différents, par un radical halogénalkoxy à chaîne droite ou à chaîne ramifiée ayant 1 à 4 atomes de carbone et 1 à 9 atomes d'halogènes identiques ou différents, et/ou par un radical halogénalkylthio à chaîne droite ou à chaîne ramifiée ayant 1 à 4 atomes de carbone et 1 à 9 atomes d'halogènes identiques ou différents, ou bien

$R^1$ et $R^2$ forment conjointement avec l'atome d'azote auquel ils sont liés, un hétérocycle saturé pentagonal à heptagonal, l'hétérocycle pouvant contenir un autre hétéroatome et pouvant être substitué une ou plusieurs fois, identiquement ou différemment, part un radical alkyle à chaîne droite ou à chaîne ramifiée ayant 1 à 4 atomes de carbone, un radical hydroxyalkyle à chaîne droite ou à chaîne ramifiée ayant 1 à 4 atomes de carbone, un radical alcanoyle à chaîne droite ou à chaîne ramifiée ayant 1 à 4 atomes de carbone dans la partie alcane et/ou par un radical alcanoyloxyalkyle ayant 1 à 4 atomes de carbone dans la partie alcane et 1 à 4 atomes de carbone dans la partie oxyalkyle,

ainsi que leurs seurs d'addition d'acides et leurs complexes de sels métalliques.

2. Procédé de production d'azolylméthylcarbinols substitués de formule

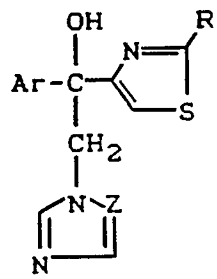

(I)

dans laquelle

Ar, R et Z ont les définitions indiquées dans la revendication 1,

ainsi que de leurs sels d'addition d'acides et de leurs complexes de sels métalliques, caractérisé en ce qu'on fait réagir des 2-bromoéthanols substitués de formule

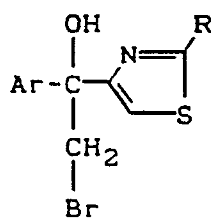

(II)

dans laquelle

Ar et R ont la définition indiquée ci-dessus, avec des azoles de formule

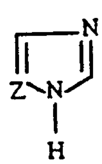

(III)

dans laquelle

Z a la définition indiquée ci-dessus,

le cas échéant en présence d'un diluant et en la présence éventuelle d'une substance auxiliaire de réaction ainsi que, le cas échéant, en présence d'un catalyseur de transfert de phase, puis on additionne éventuellement un acide ou un sel métallique.

3. Compositions fongicides et régulatrices de croissance des plantes, caractérisées par une teneur en au moins un azolylméthylcarbinol substitué de formule (I) suivant la revendication 1 ou en un sel d'addition d'acide ou en un complexe de sel métallique d'un azolylméthylcarbinol substitué de formule (I).

4. Utilisation d'azolylméthylcarbinols substitués de formule (I) suivant la revendication 1 ou de leurs sels d'addition d'acides et de leurs complexes de sels métalliques pour combattre des champignons de même que pour influencer la croissance de plantes.

5. Procédé pour combattre des champignons ainsi que pour influencer la croissance de plantes, caractérisé en ce qu'on applique des azolylméthylcarbinols substitués de formule (I) suivant la revendication 1 ou leurs sels d'addition d'acides ou leurs complexes de sels métalliques sur les plantes et/ou sur leur milieu.

6. Procédé de préparation de compositions fongicides et régulatrices de croissance des plantes, caractérisé en ce qu'on mélange des azolylméthylcarbinols substitués de formule (I) suivant la revendication 1 ou leurs sels d'addition d'acides ou leurs complexes de sels métalliques avec des diluants et/ou des agents tensioactifs.

7. 2-brométhanols substitués de formule

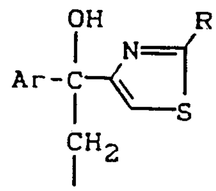

(II)

dans laquelle

Ar et R ont les définitions indiquées dans la revendication 1.

8. Procédé de production de 2-brométhanols substitués de formule

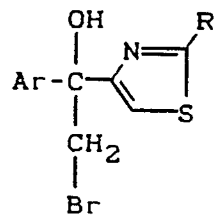

(II)

dans laquelle

Ar et R ont les définitions indiquées ci-dessus,

caractérisé en ce qu'on fait réagir des dibromacyloïnes de formule

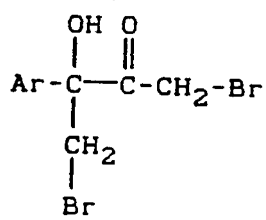

(IV)

dans laquelle

Ar a la définition indiquée ci-dessus,

avec des thiocarboxamides de formule

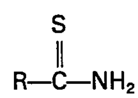

(V)

dans laquelle

R a la définition indiquée ci-dessus,

tout d'abord en présence d'une base ainsi qu'en présence d'un diluant, puis on le fait réagir en présence d'un acide de même qu'en présence d'un diluant.

**Claims**

1. Substituted azolylmethylcarbinols of the formula

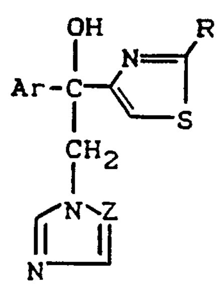

(I)

in which

Ar represents phenyl which can be monosubstituted or polysubstituted by identical or different

substituents from the series comprising halogen, straight-chain or branched alkyl having 1 to 4 carbon atoms, straight-chain or branched alkoxy having 1 to 4 carbon atoms, straight-chain or branched alkylthio having 1 to 4 carbon atoms, straight-chain or branched halogenalkyl having 1 to 4 carbon atoms and 1 to 9 identical or different halogen atoms, straight-chain or branched halogenalkoxy having 1 to 4 carbon atoms and 1 to 9 identical or different halogen atoms, and/or by straight-chain or branched halogenoalkyltho having 1 to 4 carbon atoms and 1 to 9 identical or different halogen atoms,

Z represents nitrogen or a CH group, and

R represents straight-chain or branched alkyl having 1 to 6 carbon atoms, straight-chain or branched dialkylaminoalkyl having 1 to 4 carbon atoms in each alkyl group, straight-chain or branched alkenyl having 3 to 6 carbon atoms, straight-chain or branched alkinyl having 3 to 6 carbon atoms, straight-chain or branched alkoxyalkyl having 1 to 6 carbon atoms in the alkyl moiety and 1 to 6 carbon atoms in the alkoxy moiety, or

R represents aralkyl having 1 to 4 carbon atoms in the alkyl moiety and 6 to 10 carbon atoms in the aryl moiety, where the aryl moiety can be monosubstituted or polysubstituted by identical or different substituents from the series comprising halogen, cyano, nitro, phenyl, straight-chain or branched alkyl having 1 to 4 carbon atoms, straight-chain or branched alkoxy having 1 to 4 carbon atoms, straight-chain or branched alkylthio having 1 to 4 carbon atoms, straight-chain or branched halogenoalkyl having 1 to 4 carbon atoms and 1 to 9 identical or different halogen atoms, straight-chain or branched halogenoalkoxy having 1 to 4 carbon atoms, and 1 to 9 identical or different halogen atoms, and/or by straight-chain or branched halogenoalkylthio having 1 to 4 carbon atoms and 1 to 9 identical or different halogen atoms, or by a five- or six-membered nitrogen heterocyclic ring, linked via N, which can optionally contain further hetero atoms and which can be monosubstituted or polysubstituted by identical or different substituents from the series comprising straight-chain or branched alkyl having 1 to 4 carbon atoms, straight-chain or branched alkoxycarbonyl having 1 to 4 carbon atoms in the alkoxy moiety and/or by alkylcarbamoyl having 1 to 4 carbon atoms in the alkyl moiety, or

R represents aryloxyalkyl having 1 to 4 carbon atoms in the alkyl moiety and 6 to 10 carbon atoms in the aryl moiety, where the aryl moiety can be monosubstituted or polysubstituted by identical or different substituents from the series comprising halogen, cyano, nitro, phenyl, straight-chain or branched alkyl having 1 to 4 carbon atoms, straight-chain or branched alkoxy having 1 to 4 carbon atoms, straight-chain or branched alkylthio having 1 to 4 carbon atoms, straight-chain or branched halogenoalkyl having 1 to 4 carbon atoms and 1 to 9 identical or different halogen atoms, straight-chain or branched halogenoalkoxy having 1 to 4 carbon atoms and 1 to 9 identical or different halogen atoms, and/or by straight-chain or branched halogenoalkylthio having 1 to 4 carbon atoms and 1 to 9 identical or different halogen atoms, or by a five- or six-membered nitrogen heterocyclic ring, linked via N, which can optionally contain further hetero atoms and which can be monosubstituted or polysubstituted by identical or different substituents from the series comprising straight-chain or branched alkyl having 1 to 4 carbon atoms, straight-chain or branched alkoxy-carbonyl having 1 to 4 carbon atoms in the alkoxy moiety and/or by alkylcarbamoyl having 1 to 4 carbon atoms in the alkyl moiety, or

R represents aryl having 6 to 10 carbon atoms, where the aryl radical can be monosubstituted or polysubstituted by identical or different substituents from the series comprising halogen, cyano, nitro, phenyl, straight-chain or branched alkyl having 1 to 4 carbon atoms, straight-chain or branched alkoxy having 1 to 4 carbon atoms, straight-chain or branched alkylthio having 1 to 4 carbon atoms, straight-chain or branched halogenoalkyl having 1 to 4 carbon atoms and 1 to 9 identical or different halogen atoms, straight-chain or branched halogenoalkoxy having 1 to 4 carbon atoms and 1 to 9 identical or different halogen atoms and/or by straight-chain or branched halogenoalkylthio having 1 to 4 carbon atoms and 1 to 9 identical or different halogen atoms, by a five- or six-membered nitrogen heterocyclic ring, linked via N, which can optionally contain further hetero atoms and which can be monosubstituted or polysubstituted by identical or different substituents from the series comprising straight-chain or branched alkyl having 1 to 4 carbon atoms, straight-chain or branched alkoxycarbonyl having 1 to 4 carbon atoms in the alkoxy moiety and/or by alkylcarbamoyl having 1 to 4 carbon atoms in the alkyl moiety, or

R represents a radical of the formula

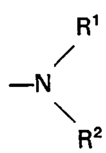

where

$R^1$ represents hydrogen, straight-chain or branched alkyl having 1 to 6 carbon atoms, straight-chain or branched alkenyl having 2 to 6 carbon atoms, straight-chain or branched alkinyl having 2 to 6 carbon atoms or cycloalkyl having 3 to 7 carbon atoms, and

$R^2$ represents hydrogen, straight-chain or branched alkyl having 1 to 16 carbon atoms, straight-chain or branched alkoxyalkyl having 1 to 4 carbon atoms in the alkyl moiety and 1 to 4 carbon atoms in the alkoxy moiety, straight-chain or branched alkenyl having 2 to 6 carbon atoms, straight-chain or branched alkinyl having 2 to 6 carbon atoms, cycloalkyl having 3 to 7 carbon atoms, cycloalkylalkyl having 3 to 7 carbon atoms in the cycloalkyl moiety and 1 to 4 carbon atoms in the alkyl moiety, or phenyl which can be

monosubstituted or polysubstituted by identical or different substituents from the series comprising halogen, straight-chain or branched alkyl having 1 to 4 carbon atoms, straight-chain or branched alkoxy having 1 to 4 carbon atoms, straight-chain or branched alkylthio having 1 to 4 carbon atoms, straight-chain or branched halogenoalkyl having 1 to 4 carbon atoms and 1 to 9 identical or different halogen atoms, straight-chain or branched halogenoalkoxy having 1 to 4 carbon atoms and 1 to 9 identical or different halogen atoms and/or straight-chain or branched halogenoalkylthio having 1 to 4 carbon atoms and 1 to 9 identical or different halogen atoms, or

$R^2$ represents aralkyl having 1 to 4 carbon atoms in the alkyl moiety and 6 to 10 carbon atoms in the aryl moiety, it being possible for the aryl moiety to be monosubstituted or polysubstituted by identical or different substituents from the series comprising halogen, straight-chain or branched alkyl having 1 to 4 carbon atoms, straight-chain or branched alkoxy having 1 to 4 carbon atoms, straight-chain or branched alkylthio having 1 to 4 carbon atoms, straight-chain or branched halogenoalkyl having 1 to 4 carbon atoms and 1 to 9 identical or different halogen atoms, by straight-chain or branched halogenoalkoxy having 1 to 4 carbon atoms and 1 to 9 identical or different halogen atoms, and/or by straight-chain or branched halogenoalkylthio having 1 to 4 carbon atoms and 1 to 9 identical or different halogen atoms,

$R^1$ and $R^2$ together with the nitrogen atom to which they are bonded represent a saturated 5- to 7-membered heterocyclic ring, where the heterocyclic ring can contain a further hetero atom, and where the heterocyclic ring can be monosubstituted or polysubstituted by identical or different substituents from the series comprising straight-chain or branched alkyl having 1 to 4 carbon atoms, straight-chain or branched hydroxyalkyl having 1 to 4 carbon atoms, straight-chain or branched alkanoyl having 1 to 4 carbon atoms in the alkane moiety and/or by alkanoyloxyalkyl having 1 to 4 carbon atoms in the alkane moiety and 1 to 4 carbon atoms in the oxyalkyl moiety, and acid addition salts and metal salt complexes thereof.

2. Process for the preparation of substituted azolylmethyl carbinols of the formula

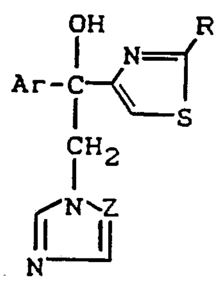

(I)

in which

Ar, R and Z have the meanings given in Claim 1, and of acid addition salts and metal salt complexes thereof

characterized in that substituted 2-bromo-ethanols of the formula

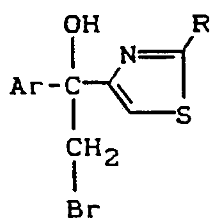

(II)

in which

Ar and R have the given meanings,

are reacted with azoles of the formula

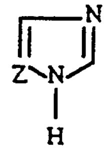

(III)

in which

Z has the abovementioned meanings,

if appropriate in the presence of a diluent and if appropriate in the presence of a reaction auxiliary and also if appropriate in the presence of a phase transfer catalyst, and the reaction product is then subjected, if appropriate, to an addition reaction with an acid or a metal salt.

3. Fungicides and plant growth-regulating agents, characterized in that they contain at least one substituted azolylmethylcarbinol of the formula (I) according to Claim 1, or an acid addition salt or a metal salt complex of a substituted azolylmethylcarbinol of the formula (I).

4. Use of substituted azolylmethylcarbinols of the formula (I) according to Claim 1, or of acid addition salts and metal salt complexes thereof, for combating fungi and for regulating plant growth.

5. Method for combating fungi and for regulating plant growth, characterized in that substituted azolylmethylcarbinols of the formula (I) according to Claim 1, or acid addition salts or metal salt complexes thereof, are applied to the plants and/or their environment.

6. Process for the production of fungicidal and plant growth-regulating agents, characterized in that substituted azolylmethylcarbinols of the formula (I) according to Claim 1, or acid addition salts or metal salt complexes thereof, are mixed with extenders and/or surface active substances.

7. Substituted 2-bromo-ethanols of the formula

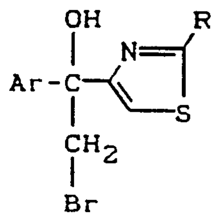

(II)

in which

Ar and R have the meanings given in Claim 1.

8. Process for the preparation of substituted 2-bromo-ethanols of the formula

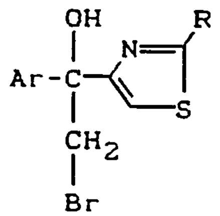

(II)

in which

Ar and R have the abovementioned meanings, characterized in that dibromoacyloins of the formula

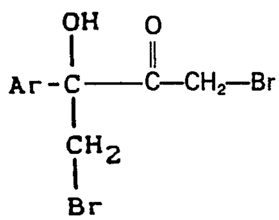

(IV)

in which

Ar has the abovementioned meaning

are reacted with thiocarboxamides of the formula

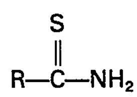

(V)

in which

R has the abovementioned meaning

initially in the presence of a base and in the presence of a diluent, and the reaction product is then reacted in the presence of an acid and in the presence of a diluent.